# EUROPEAN PATENT APPLICATION

(11) **EP 4 494 592 A1**
(43) Date of publication of application: **22.01.2025**
(21) Application number: 23185719.4
(22) Date of filing: 17.07.2023
(51) Int. Cl.: A61B 34/20, A61B 90/00, A61B 34/00, A61B 17/00, A61M 25/09

(54) **REGISTRATION OF AN IMAGING DEVICE WITH A SENSING DEVICE**

(71) Applicant: Koninklijke Philips N.V., 5656 AG Eindhoven (NL)
(72) Inventor: EKIN, Ahmet, Eindhoven (NL)
(74) Representative: Philips Intellectual Property & Standards

(57) **Abstract**

The invention relates to a registration system (13) for registering an imaging device (2) for generating two-dimensional images (7, 8) of an object (3) with a sensing device (3, 4) for acquiring sensing data indicative of a three-dimensional position and/or orientation and/or shape of the object. Besides the sensing data, object image data comprising a position and/or orientation and/or shape of the object in an image generated by the imaging device are received and/or provided in the system, wherein the system comprises a registration unit (6) configured to determine a registration between the sensing device and the imaging device based on i) the object image data, ii) the sensing data and iii) an assessment of registration confidence, wherein said determined registration is within a predetermined acceptable confidence. This can allow for a faster, but still accurate registration, particularly based on only a single image of the imaging device.

## Description

### FIELD OF THE INVENTION

The invention relates to a registration system, a registration method and a registration computer program for registering an imaging device with an elongated sensing device for acquiring sensing data indicative of a three-dimensional position and/or orientation and/or shape of an object. The invention also relates to an imaging system, an imaging method and an imaging computer program for imaging an object, and to an interventional system.

### BACKGROUND OF THE INVENTION

WO 2015/010859 A1 discloses a registration system for registering an imaging device for generating an image of an object with an optical shape sensing device for tracking the location of the object within a field of view of the imaging device by determining the position and shape of the object, the registration system comprising an object detection unit for detecting the object in the image, wherein the object detection unit is adapted to provide a representation of the object in the image, and a registration unit for determining registration parameters defining a registration of the optical shape sensing device with the imaging device, wherein the registration unit is adapted to determine the registration parameters based on the tracked location of the object in the field of view of the imaging device and on the location of the representation of the object in the image. This registration system allows for a particularly accurate registration if two or more images of the object from different directions are generated with the imaging device and used for determining the registration parameters.

### SUMMARY OF THE INVENTION

It is a goal of the invention to allow for a faster, but still accurate registration of an imaging device to an elongated sensing device for acquiring sensing data indicative of a three-dimensional position and/or orientation and/or shape of an object.

In order to achieve the goal, a first embodiment of this disclosure comprises a registration system for registering an imaging device for generating two-dimensional images of an object with an elongated sensing device arranged for acquiring sensing data indicative of a three-dimensional position and/or orientation and/or shape of the object, the registration system comprising a) a data receiving and/or providing unit configured to receive or provide i) object image data comprising a position and/or orientation and/or shape of the object in an image generated by the imaging device, and to receive or provide ii) the sensing data acquired by the sensing device, and b) a registration unit configured to determine registration parameters defining a registration between the sensing device and the imaging device based on i) the object image data, ii) the sensing data and iii) an assessment of registration confidence (or accuracy) wherein said determined registration is within a predetermined acceptable confidence (or acceptable accuracy).

Already the object image data and the sensing data can be a good basis for the registration unit to determine registration parameters defining a registration of the sensing device with the imaging device. By using a single generated image of the object as a basis for determining the registration parameters, the registration can also be carried out fast. However, it has been found that, without using at least a second image of the object generated from a different direction, the registration accuracy (or confidence) will be limited by a lack of spatial information. For instance, it has been realized that, when using only the sensing data and the object image data associated to a single two-dimensional image of the object, spatial information in a third direction, i.e., a direction out of the image plane, is missing for determining the registration parameters. Generating two or more images of the object from different directions, however, can be time-consuming. Therefore, the registration unit of the registration system presented herein is configured to determine the registration parameters based further on an assessment of registration confidence (or accuracy) wherein said determined registration is within a predetermined acceptable confidence (or acceptable accuracy). In this way, an accurate registration can be achieved even with only a single image of the object, and therefore in a faster manner.

The registration confidence can be understood as a confidence in a correctness and/or accuracy of the registration. It may be defined with respect to registration parameters defining a registration. The predetermined acceptable confidence may therefore be given in terms of a threshold value of a correctness and/or accuracy measure for registration parameters. Hence, the assessment of registration confidence may refer to a comparison of i) a correctness and/or accuracy value calculated for a set of registration parameters determined by the registration unit by use of the correctness and/or accuracy measure, with ii) the threshold value. For instance, correctness and/or accuracy values above the threshold could be associated with a sufficient registration confidence, wherein then the respective determined set of registration parameters could be accepted as a registration result.

The data receiving and/or providing unit is preferably further configured to receive and/or provide context data indicative of a position of at least one spatial reference point. The context data, in particular the position of the at least one spatial reference point, may then be used by the registration unit in addition to the object image data and the sensing data for determining the registration parameters. It has been realized that, in this way, a constraint on the possible registration parameters in the third direction can be generated, wherein the corresponding additional spatial information would particularly be missing when using only a single two-dimensional image for the registration. The received and/or provided context data can particularly be indicative of a spatial reference point whose change (or deviation) with registration parameters is a parameter for the assessment of registration confidence. Hence, the registration unit is preferably configured to use the context data, and particularly a change (or deviation) associated therewith, in the assessment of registration confidence.

A second embodiment of this disclosure comprises a registration system for registering an imaging device for generating two-dimensional images of an object with an elongated sensing device arranged for acquiring sensing data indicative of a three-dimensional position and/or orientation and/or shape of the object, the registration system comprising a) a data receiving and/or providing unit configured to receive or provide i) object image data comprising a position and/or orientation and/or shape of the object in an image generated by the imaging device, and to receive or provide ii) the sensing data acquired by the sensing device, and iii) context data indicative of a position of a spatial reference point in the image or device coordinate system, and b) a registration unit configured to determine registration parameters defining a registration between the sensing device and the imaging device based on i) the object image data, ii) the sensing data and iii) a deviation or change of the context data, when applying registration parameter, being remaining within an acceptable deviation.

In any of the above embodiments, the context data can be understood as providing information about a spatial context. To a large part, this spatial context can be expected to be fixed. In particular, the at least one spatial reference point may be chosen to regularly be stationary. Therefore, and since the registration could be regarded as mediating between a view or perception of the spatial context by the sensing device and a view or perception of the spatial context by the imaging device, changes of the spatial context, particularly the at least one spatial reference point, which could also be regarded as merely apparent changes, can be used as an indicator for a correctness and/or accuracy (or confidence) of the registration parameters and hence for a confidence in the registration.

A spatial reference point is a point that could be taken as a reference in a coordinate system, its position being typically recognizable in this system - it may be a fixed point of the system or a movable point whose motion is well-known or monitorable in the system. As an example, a reference point may be used to define the (0,0,0) point of the coordinate system.

The spatial reference points considered are preferably points of the object and/or the sensing device whose position can be determined based on the sensing data, or points in an environment in which the object moves or is to be moved whose positions are predefined or can be determined based on stored or received data regarding the environment, such as a map, for instance.

A change of various three-dimensional positions of object image data can be another parameter for the assessment of registration confidence (or accuracy). In other words, the registration unit can be configured to determine one or more positions of the object in three-dimensional space based on the object image data and carry out the assessment of registration confidence (or accuracy) based further thereon. The one or more positions of the object in three-dimensional space can correspond to a three-dimensional reconstruction of the object. A reconstruction of the object based on the object image data can be determined by use of imaging parameters and/or an imaging geometry of the imaging device, in which case the reconstruction can be understood as a virtual version of the object as viewed by the imaging device. Moreover, however, a reconstruction of the object can be determined by further using registration parameters, in which case the reconstruction can also become a virtual version of the object as expected to be viewed by the sensing device, i.e., as expected to follow from the sensing data. In particular, the registration unit can be configured to determine several reconstructions of the object based on respective several versions of registration parameters and to use changes between the several reconstructions in the assessment of registration confidence (or accuracy). For instance, the less the reconstructions change with the registration parameters, the higher the confidence (or accuracy) in a respective registration may be. If the change drops below a threshold, the respective registration parameters may be accepted to be sufficiently correct and/or accurate.

Preferably, the registration parameters are indicative of a spatial transformation between a coordinate system defined by the sensing device and a coordinate system defined by the imaging device. The spatial transformation may hence also be regarded as a coordinate transformation. The coordinate system defined by the sensing device is preferably a spatial, three-dimensional coordinate system by whose coordinates the sensing device is configured to refer to points of the object. The coordinate system defined by the imaging device is preferably a spatial, three-dimensional coordinate system by whose coordinates the imaging device is configured to refer to points in its field of view, i.e., for instance, for setting imaging parameters of a projection geometry of the imaging device. A relation between the coordinate system defined by the imaging device and any fixed, i.e. stationary, coordinate system is preferably known, particularly for all imaging directions, i.e. orientations, of the imaging device. In other words, assuming any fixed coordinate system covering both the coordinate system defined by the sensing device and the coordinate system defined by the imaging device, it is preferably known, particularly for all configurations of the imaging device, in terms of a corresponding spatial transformation how coordinates of a point in the coordinate system defined by the imaging device relate to coordinates of the point in the fixed coordinate system, whereas this will typically not be the case for the coordinate system defined by the sensing device without a registration carried out.

Preferably, the registration unit is configured to provide an expected position and/or orientation and/or shape of the object in the image based on the sensing data, given registration parameters and an imaging geometry of the imaging device. A registration without any spatial reference points could then be carried out by determining the registration parameters for which the expected position and/or orientation and/or shape of the object in the image and the position and/or orientation and/or shape of the object in the image as indicated by the object image data match, or match best. However, if the imaging device is configured to generate a two-dimensional projection image of its field of view, wherein the object image data may relate to the two-dimensional projection image, which may be preferred, then information about a position and/or orientation and/or shape of the object in a projection direction is lost through the projection. In consequence, a matching between an expected position and/or orientation and/or shape of the object in the image and the position and/or orientation and/or shape of the object in the image as indicated by the object image data will usually not be sufficient for determining accurate registration parameters, since it will usually not yield sufficient constraints on the registration parameters. Therefore, at least one spatial reference point is preferably defined in at least one of a) the three-dimensional coordinate system defined by the sensing device and/or b) the three-dimensional coordinate system defined by the imaging device (or another fixed three-dimensional coordinate system in a known relation to that defined by the imaging device), as an additional basis for determining the registration parameters. Based on this additional basis, a further matching can be carried out, wherein the further matching may be carried out in three-dimensional space instead of in the image.

In particular, the context data may further be indicative of a) a position of the at least one reference point in a three-dimensional coordinate system defined by the imaging device, and at least one of b) a position of the at least one spatial reference point in a three-dimensional coordinate system defined by the sensing device and b') a position of one or more points of the object associated with the at least one spatial reference point in a three-dimensional coordinate system defined by the sensing device. The further matching may then be carried out based on this context data.

Context data being indicative of a point P in a coordinate system A can be understood such that the context data comprise the coordinates of P in A, such that they comprise the coordinates of P in a coordinate system A' whose relation to A is known, or such that they comprise data indicating the respective coordinates. Thus, for instance, instead of the coordinates of the position of the at least one reference point in the three-dimensional coordinate system defined by the imaging device, the context data may also comprise the coordinates of the position of the at least one reference point in a fixed three-dimensional coordinate system having a known relation to that defined by the imaging device.

The one or more points of the object associated with the at least one spatial reference point can be determined based on a respective spatial reference point. For instance, all points of the object may be considered for the further matching which satisfy a predetermined relation with respect to at least one of the one or more spatial reference points. In an embodiment where the object is elongated such that it can be represented by a plurality of ordered points, the predetermined relation may be defined such that all points of the object beyond a determined reference point are considered for the further matching.

The two matchings carried out for the registration, which can also take the form of a combined matching, may be carried out with reference to respective deviations. In particular, the registration unit may be configured to determine the registration parameters based on i) a first deviation determined based on the object image data and the sensing data, and on ii) at least one second deviation determined based on the context data. The at least one second deviation can be computed, for instance, in the coordinate system defined by the imaging device or the fixed coordinate system related thereto, by transforming the positions referred to above by b) and b') using respective registration parameters.

The at least one spatial reference point is preferably fixed, i.e. stationary. Then, it can be expected that also the coordinates of the at least one spatial reference point in any fixed, i.e. stationary, coordinate system will be fixed, i.e. constant, and this expectation can serve as a constraint in the determination of the registration parameters. It has been found that this constraint can particularly be implemented by a use of reference registration parameters, which may be previously determined registration parameters. The reference registration parameters are preferably known or assumed to have been sufficiently accurate.

Accordingly, it may generally be preferred that the registration parameters determined by the registration unit are updated registration parameters defining an updated registration of the sensing device with the imaging device after a previous registration of the sensing device with the imaging device defined by previous registration parameters.

Determining updated registration parameters can be referred to as a re-registration. Reregistrations may be necessary, for instance, if the sensing device has been moved accidently, such that a spatial relationship between the sensing device and the imaging device that was present when a previous registration was carried out has changed.

The registration unit can be configured to determine the updated registration parameters based on the previous registration parameters and on the context data, i.e. in addition to the object image data and the sensing data. In particular, the updated registration parameters may be determined based on the previous registration parameters and a position of the at least one spatial reference point in a coordinate system defined by the sensing device, i.e. in addition to the position and/or orientation and/or shape of the object indicated by the sensing data and the position and/or orientation and/or shape of the object in the image indicated by the object image data. Yet more specifically, the second deviation indicated above may be determined based on i) the context data and ii) the previous registration parameters.

The registration unit is preferably configured to maintain a registration history of registration parameters, such as in a storage unit of the registration system. For instance, for each registration carried out, the registration unit can be configured to add the respective determined registration parameters to the registration history. Moreover, the registration unit is preferably configured to add, together with each of the respective registration parameters, an associated registration type and an associated registration accuracy (or confidence) to the registration history. The registration types may particularly indicate whether the respective registration parameters were determined based on a single image or based on two or more images from different directions.

Assuming the last time registration parameters were determined is not too long ago, i.e. not too far from a current time at which updated registration parameters are to be determined, it can be expected that the registration parameters will not have changed too much, i.e. that the updated registration parameters will not be too far from the last determined registration parameters. This may particularly be the case if, for instance, the imaging directions associated with the respective times are not too different. Accordingly, for updating the registration parameters, for instance, the registration unit may be configured to choose the chronologically last registration parameters in the registration history for use as previous registration in determining the updated registration parameters. The chronologically last determined registration parameters could also be regarded as the current registration parameters. Hence, an access to the registration history is in this case not actually necessary. However, if the last determined registration parameters, i.e. the current registration parameters, were determined based on a single image of the object, they may not necessarily be "trusted", may be too far from the updated registration to be determined and can therefore not serve well as reference registration parameters. The registration unit may therefore be configured to choose the chronologically last determined, i.e. the current, registration parameters for use in determining the updated registration parameters only if the associated registration accuracy (or confidence) indicated by the registration history is above a predefined threshold or has been accepted by a user approving an associated registration result. Otherwise, the registration unit may be configured to choose the chronologically last registration parameters from the stored registration history which were determined based on at least two images of the object from different directions. Since the last time this "two-view" type of registration was used might lie further in the past, in this case the registration history is actually used. The process of choosing the previous, "reference" registration parameters to be used for determining the updated registration parameters may also be viewed as an initialization for the registration parameter determination.

In a variant, the registration unit could be configured to store, with each set of registration parameters stored in the registration history, an associated viewing angle of the imaging device. For initializing a registration update, the registration unit can then be configured to determine a current viewing angle of the imaging device, search the registration history for registration parameters stored for the same view and, if such registration parameters are found, use them as reference registration parameters for the registration update.

Based on the chosen previous registration parameters and coordinates of the spatial reference point in the coordinate system defined by the sensing device, coordinates of the spatial reference point in a coordinate system defined by the imaging device can be determined by the registration unit. Furthermore, based on a registration of the imaging device with respect to a fixed further coordinate system, which is generally known in order to, for instance, be able to accurately control the imaging device to acquire images of a region of interest from predefined directions, the coordinates of the spatial reference point in the coordinate system defined by the imaging device can be transformed into coordinates of the spatial reference point in the fixed further coordinate system. The same, "two-step" mapping can be made using any possible updated registration parameters instead of the previous, "reference" registration parameters, wherein the two resulting sets of coordinates of the spatial reference point in the fixed further coordinates may be required to be as equal as possible to each other for constraining the updated registration parameters, wherein a degree of equality may be required for predetermining an acceptable level or registration confidence (or accuracy).

The registration unit can also be configured to store, for each respective set of registration parameters determined, the coordinates of the spatial reference point in the fixed coordinate system in the registration history, wherein these coordinates may be determined based on the respectively determined registration parameters. For updating the registration parameters, the registration unit may then be adapted to directly retrieve these coordinates from the registration history, without using the corresponding previous registration parameters. Accordingly, the "two-step" mapping indicated above is optionally carried out only for any registration parameters considered as possible updated registration parameters.

The coordinates of the spatial reference point in the coordinate system defined by the sensing device may be predefined, particularly if the spatial reference point is fixed. Then, the same coordinates of the spatial reference point in the coordinate system defined by the sensing device can be assumed for each update of the registration parameters. However, it is also possible that the registration unit is configured to determine the coordinates of the spatial reference point in the coordinate system of the sensing device for each determination of updated registration parameters. This can particularly be done based on physical properties of the object.

The at least one spatial reference point can particularly correspond to a position of a physical constraint for the object in three-dimensional space. Physical constraints for the object tend to fix physical properties of the object, and such fixed physical properties of the object can be used for determining the at least one spatial reference point to correspond to at least one point of the object. For instance, the sensing device and/or the registration unit can be configured to determine the coordinates of the at least one spatial reference point in the coordinate system defined by the sensing device.

By using context data indicative of a spatial reference point corresponding to a point of the object, no additional objects, i.e. no dedicated reference objects, need to be defined for the registration. The fixed physical property can particularly be a point of the object that is fixed in space by the physical constraint, wherein this point could be used as a spatial reference point. However, also other physical properties of the object may be fixed by physical constraints, wherein these other physical properties may be used, such as by the sensing device and/or the registration unit, to determine corresponding points of the object, which can then be used as spatial reference points for the registration. For instance, for a flexible object moving along more rigid curved structures, a curvature of the object may be fixed as it moves, wherein a point of the object where the curvature of the object is fixed may continuously change and hence be continuously re-determined as the object moves, wherein this point, which may be fixed in space, may be set as a spatial reference point for the registration. Similarly, for instance, a strain of the object, or a change thereof, at some position of the object may indicate a temperature change in an environment of the object at that position. The temperature change may be caused by a transition from one region in the environment to another region.

In fact, also the at least one spatial reference point is not necessarily fixed. In particular, the at least one spatial reference point may be fixed only for limited time intervals, and change from time to time, such as discontinuously. For instance, if the at least one spatial reference point is predefined to correspond to a point of the object, and if the object is being at least partially relocated from time to time, which may be on purpose, the relocation may result in a relocated spatial reference point. Therefore, the sensing device and/or the registration unit is optionally configured to determine a change in the at least one spatial reference point, and to consider the change in the at least one spatial reference point for determining the registration parameters.

In particular, the registration unit can be configured to determine whether the position of the physical constraint has changed, and, if the position of the physical constraint has changed, to determine the registration parameters based on the sensing data, the object image data, and the changed position of the physical constraint for the object.

Moreover, the spatial reference point can be chosen such that its coordinates in the coordinate system of the sensing device are not needed for determining the updated registration parameters. It may then just be defined with respect to the fixed coordinate system. As will be described further below, for instance, the spatial reference point can refer to an environment in which the object is to be tracked. Preferably, the environment provides a physical constraint on the object.

The object can be an extended, particularly an elongated, shapeable object. Hence, it may have a similar shape as the sensing device, which may also be shapeable. In fact, the sensing device may comprise the object, in which case the object itself is used for acquiring the sensing data. The sensing data may then additionally be indicative of a position and/or shape and/or orientation of a further object, to which the sensing device is attached.

The shape of the object may be sensed by the sensing device by sensing the position of several points of the object and/or by sensing a curvature and/or strain of the object at several points of the object. Based on the positions of the several points of the object, also orientations of the object at the several points may be determined, even though orientations of the object may not be directly acquired by the sensing device, i.e. may not be directly part of the sensing data. A position of the object, as indicated by the sensing data acquired by the sensing device, may hence also be understood as referring to a location, but not also to an orientation. The positions of the object indicated by the sensing data can thus be referred to, for instance, in terms of coordinates of a spatial three-dimensional coordinate system defined by the sensing device. By acquiring the sensing data over time and determining respective positions and/or orientations and/or shapes of the object associated with respective times, the position and/or orientation and/or shape of the object can be tracked. The sensing device can therefore also be referred to as a tracking device for tracking the position and/or orientation and/or shape of the object. Moreover, tracking a position and/or orientation and/or shape of an object may generally also just be referred to as tracking the object.

In particular, the sensing device may be configured to track the position and/or orientation and/or shape of the object by optical shape sensing (OSS), wherein the registration unit may be configured to determine the registration parameters based on at least the position and/or orientation and/or shape tracked by optical shape sensing, on the position and/or orientation and/or shape of the object in the image as indicated by the object image data, and on the at least one spatial reference point. OSS allows tracking the object very accurately and in a way that can easily be handled by a user. For instance, it is not necessary to provide additional fields like electromagnetic fields for tracking the object.

The data receiving and/or providing unit is optionally configured to allow a user to add markers to the image for indicating arbitrary positions in the image at which the object is located, and to provide the object image data based on the added markers. Further details and specific embodiments regarding this manner of providing the object image data are disclosed in WO 2015/010859 A1, which is herewith incorporated herein by reference in its entirety. In WO 2015/010859 A1, object image data in the form of a representation of the object in the image are obtained by an object detection unit. In fact, the object image data can generally be referred to as being an at least abstract representation of the object, while not being limited to a visual representation of the object in the image. That said, the object image data will subsequently also be referred to as a representation of the object in, or relating to, the respective image generated by the imaging device.

Several other options for receiving and/or providing the object image data, i.e. the representation of the object in the image, exist. An option according to which less user input is needed, for instance, is disclosed in WO 2016/083275 A1, which is herewith incorporated herein by reference in its entirety as well. According to this option, the data receiving and/or providing unit could be configured to provide the object image data based on a single position of the object in the image being indicated by the user, since additionally a physical representation of the object in space is used. The physical representation of the object in space may be, or be determined based on, the sensing data, such as the tracked position and/or orientation and/or shape of the object as determined by the sensing device.

Some user input for providing the object image data may be preferred for accuracy reasons. It has been found that, for some combinations of objects and imaging modalities, only users can detect at least a part of the object in the respective image accurately. However, in principle, the data receiving and/or providing unit may also be configured to provide the object image data automatically, i.e. without any user input. For instance, automatic segmentation techniques may be used by the data receiving and/or providing unit for providing the representation of the object in the image. In particular, artificial intelligence-based segmentation techniques may be used, more particularly deep-learning-based segmentation techniques. An exemplary segmentation technique based on an artificial intelligence is disclosed, for instance, in US 2022/0101034 A1, which is herewith incorporated herein by reference in its entirety. As far as the data receiving and/or providing unit is configured to provide the object image data automatically, it may be more accurately referred to as an object detection unit.

Also an automatic segmentation of the object in the image may be assisted by a physical representation of the object in space as used, for instance, in WO 2016/083275 A1 for complementing the single user-indicated position of the object in the image. The physical representation of the object in space may be provided by the sensing device, for instance.

Hence, generally, the data receiving and/or providing unit may be configured to provide object image data corresponding to a representation of the object in the image based on a segmentation of the image into an image segment representing the object and one or more further image segments, wherein the segmentation may be assisted by one or more user-indicated positions of the object in the image as providable, for instance, via a user interface, and/or by a physical representation of the object in space as it could be provided, for instance, by the sensing device.

In a preferred semi-automatic embodiment, the data receiving and/or providing unit may be configured to initially prompt the user to indicate a tip of the object by placing a marker at a corresponding image position, and to determine further points representing the object in the image based on the user-indicated point in addition to a segmentation of the object in the image and the sensing data as provided by the sensing device. If, then, the user indicates by a corresponding user input that he/she considers the determined points accurately representing the object, the object image data representing the object that are to be used for determining the registration parameters by the registration unit may be taken to correspond to the determined points, or may be determined by the data receiving and/or providing unit or the registration unit based on the determined points. If, on the other hand, the user does not yet consider the determined points accurately representing the object, he/she may indicate a further point of the object by placing a further marker at a corresponding image position or by repositioning one of the points determined by the data receiving and/or providing unit. The data receiving and/or providing unit may subsequently re-determine the object image data based on the two user-indicated points in addition to the segmentation of the object in the image and the sensing data as provided by the sensing device. This loop of user indications of object points in the image and automatic determinations of further object points in the image based on the user indications can continue until the user indicates by a corresponding user input that he/she is satisfied. Typically, only one or two user-indicated object points in the image will be necessary, however.

As mentioned above, the image that is being used for the registration is preferably a two-dimensional projection image. As also mentioned above, additionally previous registration parameters are preferably used, such that the registration parameters being determined can be referred to as updated registration parameters. Moreover, in further preferred embodiments, the registration unit is configured to determine several versions of updated registration parameters, i.e. candidate updated registration parameters, wherein the determination of each version comprises i) calculating an assumed spatial transformation between the coordinate system defined by the sensing device and the coordinate system defined by the imaging device, and ii) calculating, based on the sensing data, a two-dimensional projection of the object on the image used for registration, i.e. the image to which the object image data relate. Preferably, the registration unit is configured to select the updated registration parameters, which shall define the registration of the sensing device with the imaging device, based on values of the above mentioned first and second deviation for the several determined versions of updated registration parameters.

In particular, the registration unit can be configured to determine the updated registration parameters to correspond to that version among the several determined versions of updated registration parameters for which a combined deviation is minimized, wherein the combined deviation increases with a) a deviation between the calculated two-dimensional projection of the object and a representation of the object in the two-dimensional projection image indicated by the object image data, and b) a deviation between a first and a second position associated with each of the at least one spatial reference point in a reference coordinate system. The reference coordinate system can be the three-dimensional coordinate system defined by the imaging device or a three-dimensional coordinate system registered to the three-dimensional coordinate system defined by the imaging device. Moreover, the first position can be a position of the at least one spatial reference point in the reference coordinate system, as determined based on i) the respective version of the registration parameters and ii) the position of the at least one spatial reference point in the coordinate system defined by the sensing device. Likewise, the second position can be a position of the at least one spatial reference point in the reference coordinate system, as determined based on i) the previous registration parameters and ii) the position of the at least one spatial reference point in a coordinate system defined by the sensing device.

As already indicated above, for a fixed spatial reference point, its coordinates in the fixed further coordinate system can be expected to stay fixed as well. This requirement is preferably used for restricting the above indicated "optimization" among the several possible versions of updated registration parameters, namely in terms of the second deviation b) in the above indicated combined deviation being minimized for finding the updated registration parameters.

The minimization of the combined deviation and hence the determination of the updated registration parameters can be carried out iteratively, i.e., by successively carrying out the calculations i) and ii) and the determinations of the combined deviations in loops, wherein, in each loop, the spatial transformation is calculated based on the combined deviation and the difference from its value determined in the previous loop.

In order to further improve a confidence (or an accuracy) in the updated registration result, an alternative registration type may be used to determine reference updated registration parameters, wherein a choice for the updated registration parameters may be made between a) the updated registration parameters resulting from the above registration type using the at least one spatial reference point and the previous registration parameters and b) the reference updated registration parameters resulting from the alternative registration type. The alternative registration type preferably does not use any spatial reference point. Moreover, the alternative registration type may be carried out as disclosed in WO 2015/010859 A1, but based on only a single image of the object acquired with the imaging device.

In particular, the registration unit may be configured to a) determine first candidate registration parameters based on i) the object image data, ii) the sensing data and iii) the context data, and to determine a first registration accuracy based on i) the object image data, ii) the sensing data and iii) the first candidate registration parameters, b) determine second candidate registration parameters based on i) the object image data and ii) the sensing data without using the context data, and to determine a second registration accuracy based on i) the object image data, ii) the sensing data and iii) the second candidate registration parameters, and c) determine the registration parameters defining the registration of the sensing device with the imaging device, i.e. the updated registration parameters, based on one or a weighted combination of the first candidate registration parameters and the second candidate registration parameters based on the first registration accuracy and the second registration accuracy. In particular, the registration parameters defining the registration of the sensing device with the imaging device, i.e. the updated registration parameters, can be chosen to correspond to one of or a weighted combination of the first candidate registration parameters and the second candidate registration parameters based on the first registration accuracy and the second registration accuracy.

The first candidate registration parameters can be determined as indicated above based on the at least one spatial reference point and the minimization of the combined deviation function. The second candidate registration parameters can be determined based on current sensing data as provided by the sensing device, and object image data indicative of a representation of the object in a current image as acquired by the imaging device. The second candidate registration parameters may particularly be determined without use of any spatial reference points as a basis, and nevertheless based on only current sensing data and object image data referring to a single current image of the object as acquired by the imaging device.

Using any set of registration parameters, and hence also for the first and the second candidate registration parameters, a corresponding in-image registration accuracy can be determined based on the sensing data and the object image data by calculating a respective two-dimensional projection of the position and/or shape and/or orientation of the object indicated by the sensing data into the image under consideration of i) the spatial transformation defined by the respective registration parameters and ii) a projection geometry used by the imaging device for generating the image. The respective projection of the object may then be compared with its representation in the image in terms of the object image data, as done for the first part a) of the above indicated combined deviation. The first and the second registration accuracy are preferably determined in this way, i.e. refer to in-image accuracies. Hence, in this case, also the registration confidence (or accuracy) could be regarded as being assessed in an "in-image" way.

In particular, it is preferred that the first registration accuracy is allowed to be less than the second registration accuracy, i.e. the first candidate registration parameters are selected as output to be provided even if the first registration accuracy is less than the second registration accuracy. This recognizes the fact that the first candidate registration parameters are found in consideration of the spatial reference point and hence may be a compromise between in-image accuracy and out-of-image accuracy. In particular, the first candidate registration parameters are preferably selected as the updated registration parameters if a) the first registration accuracy is higher than a predefined minimum registration accuracy, and b) an absolute difference between the first registration accuracy and the second registration accuracy is less than a predefined registration accuracy deviation.

A further measure to ensure a good registration accuracy concerns the object image data, particularly a corresponding representation of the object in the image. If the object image data are such that the object is not accurately represented in the image, also the registration parameters will likely not be determined accurately, since their determination is based on the object image data. As briefly discussed further above, the accuracy of the representation of the object in the image can particularly be of concern if the object image data or a corresponding representation are determined automatically, i.e. without any user-indicated positions of the object in the image, and also without any review by a user.

Therefore, it may be preferred that the registration unit is configured to further determine candidate registration parameters based on i) the object image data and ii) the sensing data, wherein the object image data are indicative of several image points and associated orientations corresponding to the object, wherein the orientations are indicative of directions of straight lines between the several image points, and to determine the updated registration parameters based further on these candidate registration parameters. While generally only preferred and not strictly necessary, in this case the object image data comprise several image points, such that the associated orientations can be defined as being indicative of directions of straight lines between the several image points. Considering orientations associated with object points as part of the object image data can allow for a more accurate registration result, particularly if some points of the object have not been received and/or provided accurately by the data receiving and/or providing unit. The registration parameters determined based also on orientations, which can be referred to as "further" registration parameters, may or may not be determined based on the at least one spatial reference point.

The registration unit may be configured to determine the updated registration parameters, for instance, by choosing between a) registration parameters determined based on the first candidate registration parameters and the second candidate registration parameters, and b) the further candidate registration parameters. The choice may be made by evaluating a predefined accuracy measure for each of the two-dimensional projections of the object calculated using the considered registration parameters to choose from. This accuracy measure may be a further accuracy measure, predefined particularly for a comparison of object projections calculated from registration parameters determined based on different types of information.

In particular, the registration unit may be configured to a) validate the object image data by determining validation registration parameters based on the sensing data and the object image data, and by determining a validation registration accuracy based on the sensing data, the object image data and the validation registration parameters, and b), if the validation registration accuracy satisfies a predefined criterion indicative of satisfactory object image data, determine first candidate registration parameters based on the sensing data, the object image data and on the at least one spatial reference point, and determine a first registration accuracy based on the sensing data, the object image data and the first candidate registration parameters. Furthermore, the registration unit is preferably configured to c) assess if the validation registration accuracy satisfies a predefined criterion indicative of an unsatisfactory representation of the object in the image, and, if so, determine further candidate registration parameters based on the sensing data, the object image data comprising several image points and associated orientations corresponding to the object, and determine a further registration accuracy based at least on the sensing data, the object image data and on the further candidate registration parameters, and d) determine the registration parameters defining the registration of the sensing device with the imaging device to correspond to one or a combination of the validation registration parameters, the first candidate registration parameters and the further candidate registration parameters based on registration accuracies associated with the respective two-dimensional projections of the object into the image.

The validation registration parameters can correspond to the second candidate registration parameters described, i.e. they can be determined in the same way, particularly without use of a spatial reference point, but nevertheless based on only a single image. Under exceptional circumstances, such as when a used spatial reference point has been moved accidentally, ignoring it alone or all spatial reference points for the registration can lead to a more accurate registration.

If, as exemplarily indicated further above, the image is a two-dimensional projection image and the registration unit is able to calculate two-dimensional projections of the object as indicated by the sensing data, the further candidate registration parameters may be determined by minimizing a further combined deviation, wherein the further combined deviation increases with a) a deviation between the calculated two-dimensional projection of the object and the representation of the object correspond to the object image data, and b) a deviation between orientations associated with the two-dimensional projection of the object and the orientations comprised by the object image data.

The sensing data can in an embodiment corresponding to a plurality of ordered positions corresponding to respective points of the object. The calculated two-dimensional projection of the object can therefore correspond to a plurality of ordered image points corresponding to respective calculated positions of the object in the image. The orientations associated with the two-dimensional projection of the object may hence be calculated by the registration unit by determining, for each two consecutive points of the plurality of image points calculated to correspond to the object in the image, an orientation of a straight line connecting the two consecutive points and assigning the orientation to, for instance, a first of the two points according to the ordering. The orientation of a straight line could also be viewed as a direction of the straight line.

In order to obtain a smoother estimate of the orientations, it can be advantageous to determine the orientation associated to any given point indicated by the object image data to correspond to an average, particularly a weighted average, over orientations determined based on more than one neighboring point. For instance, respective orientations may be determined for several pairs of respective consecutive points indicated by the object image data in a neighborhood of a given point, wherein an orientation may be associated to the given point by averaging over the several orientations.

The orientations associated with a representation of the object in the image may be determined by calculating derivatives of image intensity values in two perpendicular image directions and determining the orientations associated with the representation of the object in the image based on the derivatives. If the representation of the object in the image has been determined as a plurality of straight lines connecting user-indicated and/or automatically detected object points in the image, or as a curve fitted to the object points, the orientations associated with the representation of the object in the image may also be determined based on the orientations of the lines or the curve.

The deviation between the two sets of orientations, which could also be regarded as an orientation mismatch between the projected, calculated version of the object and its imaged version, can be calculated using an L1 or L2 distance measure. For calculating the deviation between the two sets of orientations, differences in orientation between the same points of the representation and, respectively, the projection of the object in the image may be considered as for calculating a position deviation between the projection and the representation. A position deviation is preferably calculated for determining the further deviation a) on which the combined deviation depends.

In particular, the deviation between the two sets of orientations may be combined with the deviation, i.e. the position deviation, between the calculated two-dimensional projection of the object and the representation of the object to a value of the further combined deviation. This combined value can be regarded as the "cost" to be minimized for determining the further candidate registration parameters. Since also the orientations are used as features for the registration, this type of registration can also be referred to as a registration with "mixed" features.

For combinations of imaging modalities and objects known to have a tendency not to allow for a particularly accurate automatic segmentation of the respective objects in the respective images, the above indicated validation of the provided representation of the object in the image and, if necessary, subsequent registration based on "mixed" features may be particularly preferred.

For instance, segmentation techniques based on artificial intelligence have been observed not to be particularly accurate for segmenting catheters inserted into a patient's body in X-ray images thereof. In particular, it has been observed in that case that a most distal point of the catheter, i.e. the catheter tip, is often not accurately detected in the X-ray images. That it is to say, a representation of a catheter inserted into a patient's body in an X-ray image thereof, provided by the data receiving and/or providing unit by use of an artificial intelligence-based image segmentation, may still be relatively accurate for less distal parts of the catheter, but may fail at the catheter tip, leading to a wrongly detected catheter tip in the image.

However, including directional information in addition to positional information for the registration can also be beneficial more generally. The additional directional information can increase a confidence (or accuracy) into the registration if the positional information alone is not fully trusted, such as because a complete object detection in the image is not possible with particular accuracy.

Boundary points of objects, and particularly tips of elongate objects like catheters or guidewires, are usually most difficult to detect in images. Therefore, if a registration based on an automatic segmentation of an elongate object in an image fails, i.e. leads to an insufficient registration accuracy, it may be assumed with some confidence (or accuracy) that the failure is due to a wrongly detected tip of the object. It may then be preferred that the data receiving and/or providing unit is configured to provide a new set of object image data, particularly a new representation of the object in the image, that corresponds to the previously provided one up to the tip of the object. For instance, if representing the object in the image in terms of a plurality of ordered image points, the last point, corresponding to the object tip, may be removed for providing the new representation. New registration parameters may then be determined by the registration unit based on the new object image data, particularly representation, by a matching of "mixed" features, i.e. in consideration not only of points associated with the object, but also its orientations at the respective points. Based on this new registration, the data receiving and/or providing unit may, in turn, provide corrected object image data, particularly a corrected representation of the object in the image, based on the previous, "new" object image data/representation, the position of the object tip indicated by the sensing data and the new registration parameters. In other words, with the new registration parameters, which have been determined without the object tip but with orientations, the object tip may be recovered.

Even when considering each of the above options for the registration, a user may still not be satisfied with the registration accuracy and hence may have not yet sufficient confidence (or accuracy) in the registration. If this is indicated by the user, such as by a corresponding user input to the system, or if the registration unit itself concludes a final registration accuracy below a predetermined threshold, the imaging device may be controlled to generate a second image of the object from a second viewing angle, and the registration unit may be configured to determine the registration parameters based also on the second image. Hence, if needed, a "two-view" registration may be carried out. By use of the at least one spatial reference point also in this "two-view" registration, a reduction of a necessary difference in viewing angle between the two views can be achieved. In particular, it has been observed that an accurate registration based on two images generated by the imaging device from different imaging directions can already be achieved if the imaging directions differ by less than 30 degrees.

Where the object is an interventional medical instrument, the at least one spatial reference point may particularly refer to any of i) an instrument base that is fixed in the image coordinate system (i.e., the three-dimensional coordinate system defined by the imaging device), ii) a determined insertion point through which the instrument is inserted into a patient's body, and iii) a point of a lumen in a patient's body in which the instrument is movable, the position of the point of the lumen being provided a) based on an image acquired using an imaging modality that has been pre-registered with the imaging device to be registered with the sensing device and/or b) based on several stored positions of the instrument after insertion into a patient's body. The several stored positions of the instrument can particularly refer to tracked positions or paths of the instruments, which have been determined for one or more previous interventional procedures based on corresponding sensing data, and have been translated, such as by virtue of a registration used during the one or more previous interventional procedures, to the coordinate system defined by the imaging device or a fixed coordinate system related thereto in a known way.

Each of the above five exemplary choices for the at least one spatial reference point has been found to be practical in the interventional setting, both when taken alone as well as when taken in combination, i.e. when several spatial reference points are considered of which each is chosen according to a different one of the five mentioned exemplary options. Notably, the at least one spatial reference point is not restricted to lie in the field of view of the imaging device and therefore is not necessarily visible in the image used for registration.

The interventional instrument is preferably a flexible elongate instrument like a catheter or a guidewire for a catheter. The instrument base preferably corresponds to a most proximal point of the instrument, and this point is taken as the spatial reference point. It is then not necessary to re-determine the coordinates of the spatial reference point in the coordinate system defined by the sensing device for each update of the registration parameters. The coordinate system defined by the sensing device can be such that it always assigns the same coordinates, such as its origin, to the most proximal point of the instrument.

In order to use an insertion point into a patient's body, the sensing device is preferably an OSS device comprising an optical fiber whose strain is measured for each of a plurality of positions along the fiber. For instance, the strain can be part of the sensing data that also indicate the positions along the fiber. The fiber can be attached to the guidewire, for instance. Since the strain depends on temperature and since there will be a temperature change at the insertion point, the coordinates of the insertion point in the coordinate system defined by the sensing device can be estimated by the registration unit based on the measured strains along the fiber.

Coordinates associated with a spatial reference point corresponding to a position of a point of a lumen in a patient's body as defined in the coordinate system of the sensing device can be estimated by the registration unit by matching a position and/or orientation and/or shape of the object, as indicated by the sensing data, to a position and/or orientation and/or shape of the lumen and determining the coordinates of the spatial reference point in the coordinate system defined by the sensing device to be those of a point of the instrument which is associated, according to the matching, to the position of the point of the lumen corresponding to the spatial reference point.

However, it is not necessary to explicitly determine the coordinates of a position of the lumen considered as spatial reference point in the coordinate system defined by the sensing device. In fact, it may be preferred that the registration unit is configured to transform, using a respective version of the registration parameters considered in determining the updated registration parameters, a position and/or orientation and/or shape of the instrument as determined based on the sensing data into a map of the lumen and determine the updated registration parameters based on a deviation between a position and/or orientation and/or shape of the lumen and transformed position and/or orientation and/or shape of the instrument in the map. The deviation can be defined, for instance, as an average minimum distance of the transformed position and/or orientation and/or shape to the position and/or orientation and/or shape of the lumen in the map, wherein parts of the transformed version of the instrument outside of the lumen are assigned a distance equal to zero.

If the map only covers regions inside the lumen, a minimum distance between each instrument point outside the map and a border of the map may be calculated in a coordinate system in which the map is defined and to which the coordinates of the instrument points indicated by the sensing data have been transformed, instrument points inside the map are assigned a distance of zero, and an average of the distances is calculated and considered as the deviation between the lumen and the instrument in the map. Hence, effectively a whole in-body section of the transformed version of the instrument may be used as an additional basis for determining the registration parameters. The spatial reference point can in this case be regarded as corresponding to the border of the map.

If the map covers an interior of a patient's body but not more, an in-body-section of the instrument may first be determined in the coordinate system defined by the sensing device based on the sensing data, particularly based on a localization of an in-body insertion point using strain changes indicated by the sensing data as explained further above. Only the in-body section may subsequently be transformed, using a respective version of the registration parameters considered in determining the updated registration parameters, into the coordinate system of the map. Then, the above calculation may be repeated analogously. That is to say, a minimum distance between each instrument point of the (transformed) in-body section outside the map and a border of the map may be calculated, instrument points of the (transformed) in-body section inside the map are assigned a distance of zero, and an average of the distances is calculated and considered as the deviation between the body insertion point and the instrument in the map. The spatial reference point can in this case be regarded as corresponding to the body insertion point, which will however lie again on the border of the map if the map covers the interior of the patient's body but not more.

As discussed in more detail further below, the map of the lumen may be a stored vessel map generated based on an image acquired using an imaging modality that has been pre-registered with the image device to be registered with the sensing device, or a shape occupancy map generated based on several stored positions of the instrument after insertion into a patient's body, as they would typically be indicated by sensing data acquired in previous interventional procedures.

When using several spatial reference points and associated deviations for determining the registration parameters, the registration unit may be configured to determine an average of the respective deviations associated with the several spatial reference points. That is to say, for determining the part of the combined deviation taking into account the at least one spatial reference point, which is preferably determined in the coordinate system defined by the imaging system, the table coordinate system or another coordinate system registered to any of the two, a respective deviation is determined for each of the several reference points, and an average of the several determined deviations is taken as the quantity b) to enter the combined deviation. The average may also be a weighted average, wherein the weights may be chosen by the registration unit based on predefined confidences (or accuracies) into a use of the respective spatial reference points and associated deviations for determining registration parameters.

It is a further goal of the invention to allow for an easier, but still accurate navigation of an object, particularly an interventional instrument.

In order to achieve the further goal, an imaging system for imaging an object is presented, the imaging system comprising a) an imaging device for generating an image of a region of interest, b) a sensing device for acquiring sensing data indicative of a three-dimensional position and/or orientation and/or shape of the object in the region of interest, c) a registration system as described above for determining registration parameters defining a registration of the sensing device with the imaging device, and d) a navigational image providing unit for providing a navigational image of the region of interest including a representation of the object based on the sensing data and the determined registration parameters.

The navigational image can be understood as an image of the region of interest based on which the object is to be navigated. The navigational image providing unit can be configured to first provide a navigational image, and to then determine a representation of the object in the image based on the sensing data and the determined registration parameters. The latter step can also be regarded as an identification of the object in the previously provided image. Identifying the object in the image can comprise determining one or more positions of the object in the image. Therefore, the navigational image providing unit can also be understood as comprising a position determination unit. Since the imaging device and the tracking device can be registered more accurately by virtue of the registration system based on only a single image, it is no longer necessary to acquire two or more images of the object from different directions every time a registration is carried out. Hence, a faster, but still accurate registration can be carried out. Based on the registration, the actual, physical position of the objection can be shown accurately in the navigational image, which might also be referred to as a "position image", and this image can assist in navigating the object. The faster registration therefore also allows for an overall easier, but still accurate navigation. An easier navigation may also translate to reduced navigation times.

The navigational image can particularly be a different image than the image used for determining the registration parameters, even though the navigational image may also be a two-dimensional image. For instance, the registration may be carried out based on an image generated by the imaging device at a first point in time, and the object may be shown in an image generated by the imaging device at a second point in time, wherein the second point in time lies after the first point in time. The second, "position" image may be generated from an arbitrary viewing angle, and the object may have moved between the first and the second point in time.

The navigational image is also not necessarily acquired by the imaging device generating the image used in the registration system. Then, the navigational image will typically need to be pre-registered with the imaging device. The pre-registration between the navigational image and the imaging device generating the image used for registration corresponds to a registration of this imaging device with the further imaging device used for acquiring the navigational image. By virtue of the pre-registration, it is preferably known how coordinate systems defined by the respective imaging devices relate to each other. The pre-registration may particularly be carried out based on the navigational image and an image of the region of interest generated by the imaging device used in the registration system.

In fact, optionally the navigational image is a three-dimensional image of the region of interest. Three-dimensional images can allow for a more accurate and hence safe navigation of the object. In particular, the navigational image, in which the identification unit is configured to identify the object, can be a stored three-dimensional image of the region of interest, wherein the navigational image providing unit may provide the navigational image by retrieving it from a storage unit. The navigational image may have been generated before the registration, particularly before a respective navigational task for the object has been initiated. The pre-registration of the navigational image with the imaging device used in the navigational task and to be registered with the sensing device may then be carried out at an initial step of the navigational task. For instance, a pre-interventional volumetric image of a patient anatomy may be pre-registered with an imaging device generating two-dimensional images during an interventional procedure, which are used for registration to a sensing device, once a patient has been placed in a position in which he/she is set to undergo the image-guided interventional procedure. The pre-registration can then be carried based on an image generated by the imaging device of the patient.

As indicated, the object being "sensed" or "tracked" can particularly be an interventional instrument. An easier, but still accurate navigation of an interventional instrument allows for reduced intervention times and can hence also make the interventions safer, i.e. increase patient safety, for instance. The interventional instrument can particularly be a catheter and/or a guidewire for guiding a catheter during a medical intervention.

In a preferred embodiment, the image used for the registration is a two-dimensional X-ray image, and the imaging device of the imaging system can be an X-ray imaging device that may have been pre-registered to a volumetric CT image of the region of interest. The CT image may have been generated before an intervention requiring a navigation of the interventional instrument. For navigating the object based on the CT image as navigational image, the user may then select any desired view to be rendered from the CT image, wherein the rendered view may then be displayed on a display of the imaging system. Also the stored vessel and/or shape occupancy map that, as indicated above, is optionally used for defining a spatial reference point can be generated based on the CT image.

In order to achieve the goal of reduced intervention times and/or an increased intervention safety, also an interventional system is presented, the system comprising an interventional instrument for carrying out an interventional procedure, and an imaging system for imaging the interventional instrument as described above.

In order to achieve the goal of allowing for a faster, but still accurate registration of an imaging device to an elongated sensing device for acquiring sensing data indicative of a three-dimensional position and/or orientation and/or shape of an object, also a registration method for registering an imaging device for generating two-dimensional images of an object with an elongated sensing device arranged for acquiring sensing data indicative of a three-dimensional position and/or orientation and/or shape of the object is presented, the registration method including a step of receiving and/or providing i) object image data comprising a position and/or orientation and/or shape of the object in an image generated by the imaging device, and ii) the sensing data acquired by the sensing device. The method further includes a step of determining registration parameters defining a registration between the sensing device and the imaging device based on i) the object image data, ii) the sensing data and iii) an assessment of registration confidence (or accuracy) wherein said determined registration is within a predetermined acceptable confidence (or acceptable accuracy). The registration method shares the advantages of the above described registration system.

In order to achieve the goal of allowing for a more accurate navigation of an object, particularly an interventional instrument, also an imaging method for imaging an object is presented, the imaging method comprising steps of a) generating an image of a region of interest by an imaging device, b) acquiring sensing data indicative of a three-dimensional position and/or orientation and/or shape of the object in the region of interest by a sensing device, c) determining registration parameters defining a registration of the sensing device with the imaging device by a registration method as described above, and d) providing a navigational image of the region of interest including a representation of the object based on the sensing data and the determined registration parameters. The imaging method shares the advantages of the above described imaging system.

Moreover, the invention relates to a registration computer program for registering an imaging device for generating two-dimensional images of an object with an elongated sensing device arranged for acquiring sensing data indicative of a three-dimensional position and/or orientation and/or shape of the object, the registration computer program comprising program code means for causing the registration system as described above to carry out the steps of the registration method as described above when the registration computer program is run on a computer controlling the registration system.

Likewise, the invention further relates to an imaging computer program for imaging an object, the imaging computer program comprising program code means for causing the imaging system as described above to carry out the steps of the imaging method as described when the imaging computer program is run on a computer controlling the imaging system.

It shall be understood that the registration system of claim 1, the imaging system of claim 12, the interventional system of claim 13, the registration method of claim 14 and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of an interventional system,
Fig. 2 shows schematically and exemplarily a projection image acquired by an imaging device of the interventional system, wherein the image shows an object in a region of interest,
Fig. 3 shows schematically and exemplarily coordinate systems referred to for registering a sensing device with an imaging device,
Fig. 4 shows schematically and exemplarily a registration method for registering the imaging device with the sensing device,
Fig. 5 shows schematically and exemplarily a further registration method for registering the imaging device with the sensing device,
Fig. 6 shows schematically and exemplarily a docking top for a catheter guidewire,
Fig. 7 shows schematically and exemplarily a patient anatomy including an indication of possible insertion points for the catheter into the patient's body,
Fig. 8 shows schematically and exemplarily a user interface of an imaging system of the interventional system, and
Fig. 9 shows a flowchart exemplarily illustrating an embodiment of an imaging method.

### DETAILED DESCRIPTION OF EMBODIMENTS

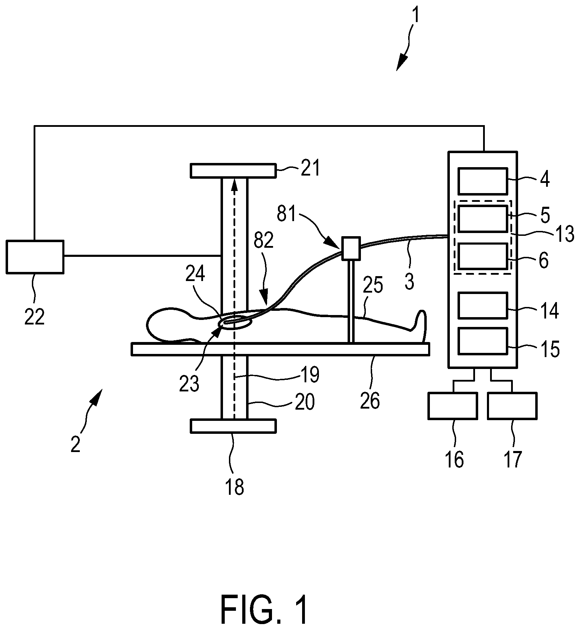

Fig. 1 shows schematically and exemplarily an embodiment of an interventional system. The interventional system 1 comprises an interventional instrument 33 for carrying out an interventional procedure. In this embodiment the interventional instrument 33 is a catheter, which is controlled by a catheter control unit 15. The catheter control unit 15 and the catheter 33 may be adapted to perform, for instance, an ablation procedure within a heart 24 of a person 25 lying on a support means like a patient table 26. However, the catheter 33 and the catheter control unit 15 can also be adapted to perform another interventional procedure. Moreover, the interventional system can also comprise another interventional instrument like a needle. In this embodiment the catheter 33 is adapted to apply a radio frequency (RF) ablation procedure, wherein the catheter control unit 50 is adapted to provide RF energy to ablation electrodes arranged at the tip 23 of the catheter 33.

For inserting the catheter 33 into the heart 24 a guidewire 3 is used, which is a further interventional instrument and which is equipped with an optical shape sensing (OSS) fiber connected to a tracking control unit 4 for determining the position and/or orientation and/or shape of the guidewire within the person 25 by OSS. For determining the location of the guidewire within the person 25, known OSS techniques can be used like the technique disclosed in US 7,772,541 B2, which is herewith incorporated by reference. The guidewire 3 equipped with the OSS fiber and the tracking control unit 4, in particular, the OSS fiber and the tracking control unit 4, can be regarded as being a sensing device arranged for acquiring sensing data indicative of a three-dimensional position and/or orientation and/or shape of an object. In particular, the locations of the guidewire 3, which collectively give rise to an orientation and a shape thereof, can be tracked in three spatial dimensions. The sensing device established by the tracking control unit 4 and the guidewire 3 being equipped with the OSS fiber will therefore subsequently be referred to as a tracking device.

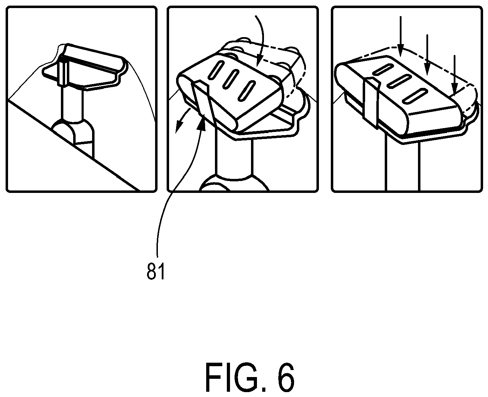

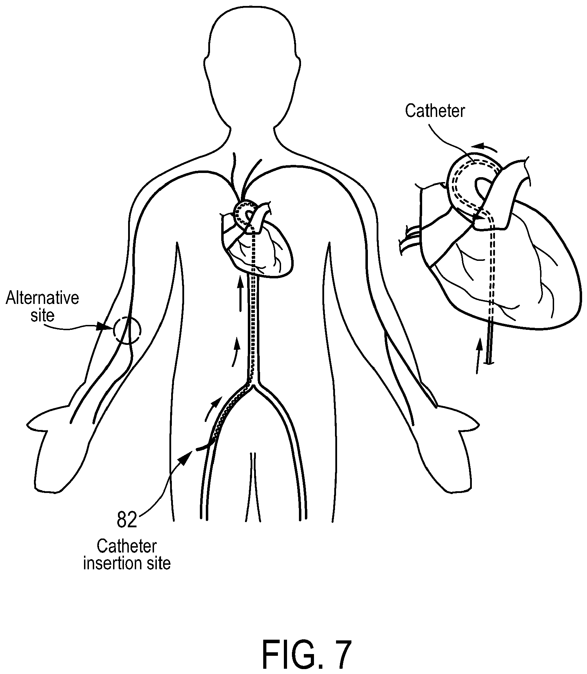

The guidewire 3 equipped with the OSS fiber is being slid through a slot of a docking top 81 mounted to the table 26, and enters the body at a point 82. The docking top 81 is shown in more detail in Fig. 6, and possible entry points are shown in Fig. 7. The catheter 33 can, in principle, also be led through the docking top 81. But it may be preferred that the catheter 33 is slid over the guidewire 3 at a more distal point along the guidewire 3. In this way, limitations to certain types of catheters 33 caused by the docking top 81 may be avoided. For sliding the catheter 33 over the guidewire 3 at a more distal point, a separate device dedicated to this purpose, which may be regarded as an adapter, can be used.

Although in this embodiment an OSS fiber is used for tracking the position and/or orientation and/or shape of the guidewire 3, the tracking device is not limited thereto. For instance, in other embodiments an electromagnetic (EM) tracking device may be used, in which case EM markers might be attached to the guidewire 3 and an EM coil system may be used for generating an EM field based on which the positions of the EM markers may be determined.

The interventional system 1 further comprises an imaging device 2 being, in this embodiment, an X-ray C-arm system. The imaging device 2 comprises an X-ray source 18 generating X-rays 19 traversing the person 25 and an X-ray detector 21 for detecting the X-rays after having traversed the person 25. The X-ray source 18 and the X-ray detector 21 are attached to a C-arm 20, which is rotatable around the person 25 for acquiring two-dimensional X-ray projection images in different projection directions. The imaging device is controlled by an imaging device control unit 22, which receives detection values from the X-ray detector 21 and generates the two-dimensional projection images based on the received detection values. The two-dimensional projection images are in this embodiment provided to an object detection unit 5 for detecting the guidewire in the two-dimensional projection images. The object detection unit 5 has two modes for doing so.

In a first, semiautomatic mode, the object detection unit 5 is configured to a) allow a user to add markers to a two-dimensional projection image for indicating arbitrary positions in the two-dimensional projection image, at which the guidewire is located, and b) provide a representation of the guidewire in the two-dimensional projection image based on the added markers. The object detection unit 5 preferentially provides a graphical user interface, which allows a user to add the markers to the two-dimensional projection image by using an input unit 16 like a keyboard, a computer mouse, a touch screen, et cetera, and a display 17.

In a second, automatic mode, the object detection unit 5 is configured to provide a representation of the guidewire in the two-dimensional projection images by use of an artificial intelligence that has been trained to segment guidewires in medical X-ray images. For instance, the artificial intelligence used by the object detection unit 5 may be trained as disclosed in US 2022/0101034 A1, which is herewith incorporated herein by reference again. The segmentation is complemented by the tracked position and/or orientation and/or shape of the guidewire in order to increase an accuracy of the representation of the guidewire provided by the object detection unit 5.

An output of the tracking control unit 4 and the object detection unit 5 is collected by a data receiving and/or providing unit and provided for any further processing needed for the registration between the imaging device 2 and the tracking device 3, 4. The data receiving and/or providing unit could therefore, in this embodiment, also be understood as comprising, on the one hand, an interface to the tracking device 3, 4 and, on the other hand, an image processing unit and/or user input unit corresponding to the object detection unit 5. The output by the object detection 5, which in this embodiment corresponds to a representation of the guidewire 3 in an image, can be understood as object image data comprising a position and/or orientation and/or shape of the guidewire 3 in the image.

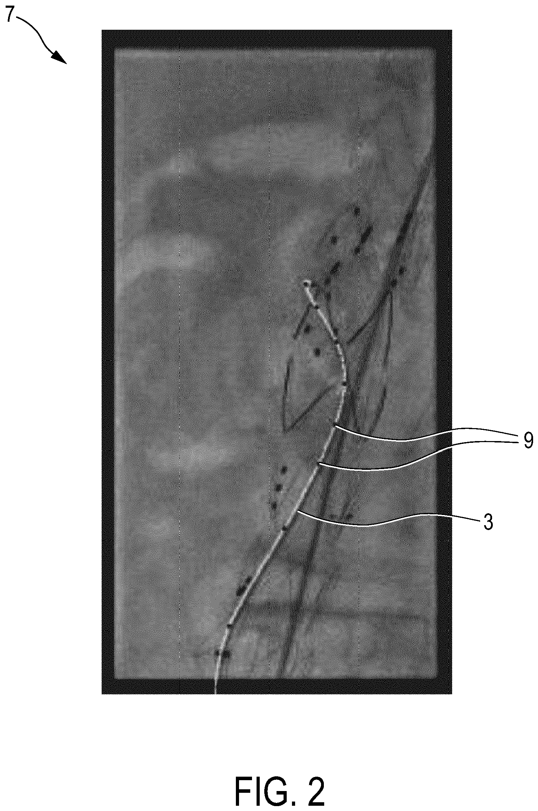

Fig. 2 shows schematically and exemplarily a two-dimensional projection image 7 that has been acquired with the X-ray C-arm system being, in this embodiment, the imaging device 2, and in which the guidewire 3 can be seen by virtue of a representation thereof in terms of a white line, which has been determined by the object detection unit 5 and overlaid on the projection image 7. Without the representation being determined, the guidewire 3 would appear dark. In this case, the object detection unit 5 has determined the representation of the guidewire 3 automatically based on automatically determined markers 9 along the guidewire 3 in the image 7, wherein the markers 9 have been determined based on a segmentation of the image 7 and a detection of the guidewire 3 using sensing data acquired by the tracking device 3, 4. The representation has been determined based on the markers 9 by computing a minimum cost path between the image points corresponding to the markers 9. The minimum cost path may be defined with respect to an elongatedness measure associating a cost to deviations of paths away from image points corresponding to the guidewire 3 according to the segmentation.

While, in the shown embodiment, the markers 9 and the representation of the guidewire 3 in the image 7 have all been determined fully automatically, this can be different in other embodiments.

For instance, one or more of the markers 9 may alternatively be added to the two-dimensional projection image 7 by the user. In particular, as described in more detail further below, the user may be prompted to indicate the tip of the guidewire 3, and using this as additional basis the object detection unit 5 may generate other points along the guidewire automatically. In case one of those generated points is wrong, the user may add another point. Based on this as a further additional basis, i.e., based on the two points of the guidewire 3 indicated by the user as an additional basis, the object detection unit 5 may generate another set of points along the guidewire 3 automatically. This loop of successively more user-indicated guidewire points and automatic generation of further guidewire points can continue until the user is satisfied. Often, however, the indication of the guidewire tip will be enough.

Moreover, a representation of the guidewire 3 could alternatively be determined by the object detection unit 5 based on the added markers 9 by, for instance, just connecting the markers 9 by multiple line segments or fitting a curve to the added markers 9.

As indicated in Fig. 1, the interventional system 1 further comprises a registration unit 6 for determining registration parameters defining a registration of the tracking device 3, 4 with the imaging device 2, wherein the registration unit 6 is configured to determine the registration parameters based on the tracked position and/or orientation and/or shape of the guidewire 3 in the field of view of the imaging device 2, on the image positions of the representation of the guidewire 3 in the two-dimensional projection image 7, and on at least one spatial reference point.

In this embodiment, a single spatial reference point is used, wherein the single spatial reference point is associated with the slot of the docking top 81 attached to the table 26 on which the patient 25 lies. Since the guidewire 3 and optionally also the catheter 33 are being advanced and retracted through the slot of the docking top 81, it is also referred to as a launch base. A most proximal tracked point of the guidewire 3 may be defined by the launch base. In a particular example, the spatial reference point refers to the most proximal point of the guidewire 3 being tracked by the tracking device 3, 4.

While in this embodiment a single spatial reference point associated with a docking top for the guidewire 3 and the catheter 33 is used for determining the registration parameters, more than one spatial reference point may be used in other embodiments, wherein none of the spatial reference points is necessarily related to a docking top. Using more than one spatial reference point for determining the registration parameters can further increase a registration accuracy. This will be described in more detail further below.

Furthermore, in this embodiment the registration parameters determined by the registration unit 6 are updated registration parameters defining an updated registration of the tracking device 3, 4 with the imaging device 2 after a previous registration of the tracking device 3, 4 with the imaging device 2 defined by previous registration parameters. For instance, while the interventional system 1 is in use, such as while a radiofrequency ablation procedure is being carried out, the registration unit 6 may be configured to determine updated registration parameters one or more times in order to ensure that a good registration accuracy is maintained. In particular, updated registration parameters may be determined upon request by a user via a corresponding user input.

Optionally, if a re-registration is requested by a user for some imaging direction of the imaging device 2, i.e., when the C-arm 20 has a certain orientation, it may be checked by the registration unit 6 whether registration parameters have already been determined previously for this imaging direction, i.e., if the C-arm 20 already had the same orientation at an earlier time and registration parameters were determined at the earlier time. If so, the registration parameters from the earlier time may be taken as updated registration parameters by the registration unit 6. According to another option, the registration parameters from the earlier time may be taken as reference registration parameters in a registration update. Generally, however, a particular dependence of the registration parameters on the imaging direction is not expected, such that a use of stored registration parameters for a current imaging direction in a requested re-registration is not necessary. In particular, any other previous registration parameters may in principle be used as reference registration parameters.

Preferably, the registration unit 6 is configured to determine the updated registration parameters based on the respective previous registration parameters and on the position of the spatial reference point in a coordinate system defined by the tracking device 3, 4. Moreover, the registration unit 6 is in this embodiment configured to determine several versions of the updated registration parameters. Each of the several versions of the updated registration parameters is determined by first calculating an assumed spatial transformation between the coordinate system defined by the tracking device 3, 4 and a coordinate system defined by the imaging device 2, and by then calculating a two-dimensional projection of the tracked guidewire 3 under consideration of a projection geometry used by the imaging device 2 for generating the two-dimensional projection image 7, wherein the registration unit 6 is configured to determine the updated registration parameters to correspond to that version among the several determined versions of updated registration parameters for which a combined deviation function is minimized. The combined deviation function is defined such that it combines a deviation in image space with a deviation in physical space. Preferably, the combined deviation function is defined such that it increases with each of a) a deviation between the respective calculated two-dimensional projection of the tracked guidewire 3 and the representation of the guidewire 3 in the two-dimensional projection image, and b) a deviation between a first and a second position in a reference coordinate system, wherein the reference coordinate system is the three-dimensional coordinate system defined by the imaging device 2 or a three-dimensional coordinate system registered to the three-dimensional coordinate system defined by the imaging device 2. The first position is a position of the at least one spatial reference point in the reference coordinate system as determined based on the respective version of the registration parameters and the position of the at least one spatial reference point in the coordinate system defined by the tracking device 3, 4, and the second position is a position of the at least one spatial reference point in the reference coordinate system as determined based on the previous registration parameters and the position of the at least one spatial reference point in a coordinate system defined by the tracking device 3, 4.

In an example, the combined deviation corresponds to a weighted average of the two mentioned deviations, i.e. can be written Combined_Distance = w1 * Image_Distance + w2 * Context_Distance, where Image_Distance refers to the deviation a) between the calculated two-dimensional projection of the tracked guidewire 3 and the representation of the guidewire 3 in the two-dimensional projection image, Context_Distance refers to the deviation b) between the spatial reference point as determined based on the respective version of the registration parameters and the spatial reference point as determined based on the previous registration parameters, and where w1 and w2 are predetermined, fixed weights. In particular, w1 = w2 = 1 may be used, in which case the weighted average could also be regarded as an ordinary sum.

By use of the combined deviation for finding the version of the registration parameters used for updating, it can be tried to both a) align the projected version of the tracked guidewire 3 with the representation of the guidewire 3 determined based on the projection image, and to b) find an updated registration by which the spatial reference point, as determined in the coordinate system of the tracking device 3, 4, is mapped to coordinates of the coordinate system defined by the imaging device 2 that are the same or at least similar as for the previous registration.

For aligning the projected version of the tracked guidewire 3 with the representation of the guidewire 3 determined based on the projection image, i.e., for determining the quantity Image_Distance, it can be sufficient and hence computationally efficient, and/or even increase a robustness of the registration, to consider only parts of the projection and/or the representation. For instance, distances between the projection and the representation may be determined only for selected points of the guidewire 3, i.e., only for selected points of the projection and the representation. In fact, the representation of the guidewire 3 itself may only correspond to the selected points. The selected points may be distributed along the guidewire 3, for instance, as seen in Fig. 2 in terms of the markers 9 along the representation of the guidewire 3. On the other hand, the projection is preferably computed entirely, i.e., not only for selected points. This is because a useful selection of particular points of the tracked shape of the guidewire 3 for the projection and hence the subsequent determination of the quantity Image_Distance could reasonably be made only based on an already known "1-to-1 link" between image points corresponding to the guidewire 3 and points of the tracked shape of the guidewire 3. Such a "1-to-1 link", however, is yet what is aimed at via the registration.

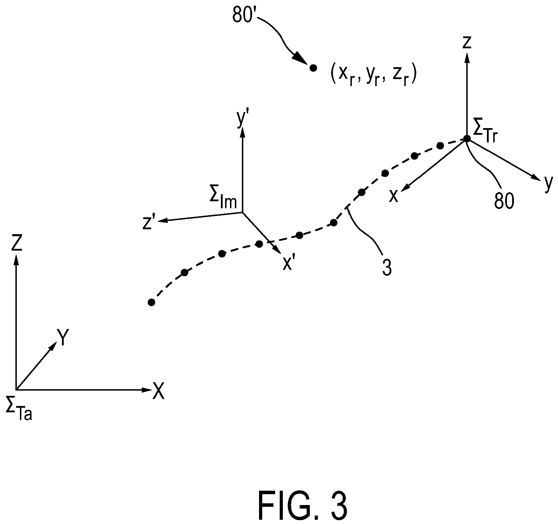

Fig. 3 shows schematically and exemplarily a coordinate system Σ_{Tr} in which points of the guidewire 3 are being tracked with the control unit 4, a coordinate system Σ_{Im} defined by the imaging device 2, and the points of the guidewire 3. When defining the registration in terms of a spatial transformation from the coordinate system Σ_{Tr} to the coordinate system Σ_{Im}, coordinates (x, y, z) associated to the points of the guidewire 3 in the coordinate system Σ_{Tr} can be mapped, by the spatial transformation, to coordinates (x', y', z') associated to respective same points of the guidewire 3 in the coordinate system Σ_{Im}. Fig. 3 further shows a coordinate system Σ_{Ta} defined by the table 26 on which the patient 25 is lying. The imaging device 2 is already calibrated, such that a spatial transformation from the coordinate system Σ_{Im} to the coordinate system Σ_{Ta} is known for all imaging directions, i.e. all viewing angles, of the imaging device 2. Hence, coordinates (x', y', z') associated to points of the guidewire 3 in the coordinate system Σ_{Im} can be mapped to coordinates (X, Y, Z) associated to respective same points of the guidewire 3 in the coordinate system Σ_{Ta}.

Fig. 3 further shows schematically two examples 80, 80' for a spatial reference point that can be used for determining the registration parameters defining the spatial transformation between Σ_{Tr} and Σ_{Im}. In the first example 80, the spatial reference point is chosen to be the first, i.e. most proximal, tracked point of the guidewire 3, which refers to the slot of the docking top 81 currently in use. In this embodiment the origin of the coordinate system Σ_{Tr} is placed such that it coincides with the first tracked point of the guidewire 3. Hence, the spatial reference point 80 is assigned coordinates (x, y, z) = (0, 0, 0) in the coordinate system Σ_{Tr}. In another example, the spatial reference point 80 could be chosen to correspond to the point of the guidewire 3 where it enters the body of the person 25, whose coordinates in the (x, y, z) in the coordinate system Σ_{Tr} will change as the guidewire 3 moves, but may be estimated based on a strain of the OSS fiber with which the guidewire 3 is equipped.

According to a further choice 80', the spatial reference point could also not coincide with any point of the guidewire 3. In principle, the spatial reference point 80' could be placed anywhere in the interventional setting. In particular, the spatial reference point 80' could be chosen based on a map of a lumen in the body of the person 25 into which the guidewire 3 and the catheter 33 are inserted. As indicated in Fig. 3, the coordinates of the spatial reference point 80' could generally be referred to as (x, y, z) = (Xr, yᵣ, Zr).

Assuming the spatial reference point 80 corresponding to the first tracked point of the guidewire 3 and being associated with the coordinates (0, 0, 0) in the coordinate system Σ_{Tr}, and denoting the spatial transformation from Σ_{Tr} to Σ_{Im} by T_{Tr→Im} and the spatial transformation from the coordinate system Σ_{Im} to the coordinate system Σ_{Ta} by T_{Im→Ta}, the coordinates associated with the spatial reference point 80 in the coordinate system Σ_{Ta} can be written (X, Y, Z) = T_{Im→Ta} [T_{Tr→Im}[(0, 0, 0)]]. Each version of the registration parameters defines a potentially different transformation T_{Tr→Im} and hence potentially different transformed coordinates (X, Y, Z) of the spatial reference point 80 in the coordinate system Σ_{Ta}. For finding the updated registration parameters, however, it is preferably required that these coordinates of the spatial reference point 80 in the coordinate system Σ_{Ta} deviate as little as possible from the corresponding coordinates resulting from a use of the transformation T_{Tr→Im} defined by respective previous registration parameters. The size of the deviation is determined by the minimum of the above-mentioned combined deviation function. The respective previous registration parameters considered for this purpose may be the chronologically last registration parameters determined or other "reference" registration parameters. In particular, the used "reference" registration parameters may be chosen to be registration parameters determined based on at least two images acquired by the imaging device 2 from different directions, wherein these registration parameters may be retrieved from a registration history maintained by the registration unit 6.

With the spatial reference point chosen to correspond to the first tracked position of the guidewire 3 fixed by the docking top 81, not only its coordinates (x, y, z) = (0, 0, 0) in Σ_{Tr} are fixed, but, as long as the slot of the docking top 81 being in use is not changed and the docking top 81 is not moved, also its coordinates in the coordinate system Σ_{Ta}, and hence these coordinates can be once determined and stored. The above requirement could then be rephrased such that the coordinates (X, Y, Z) should deviate as little as possible from the coordinates once determined and stored.

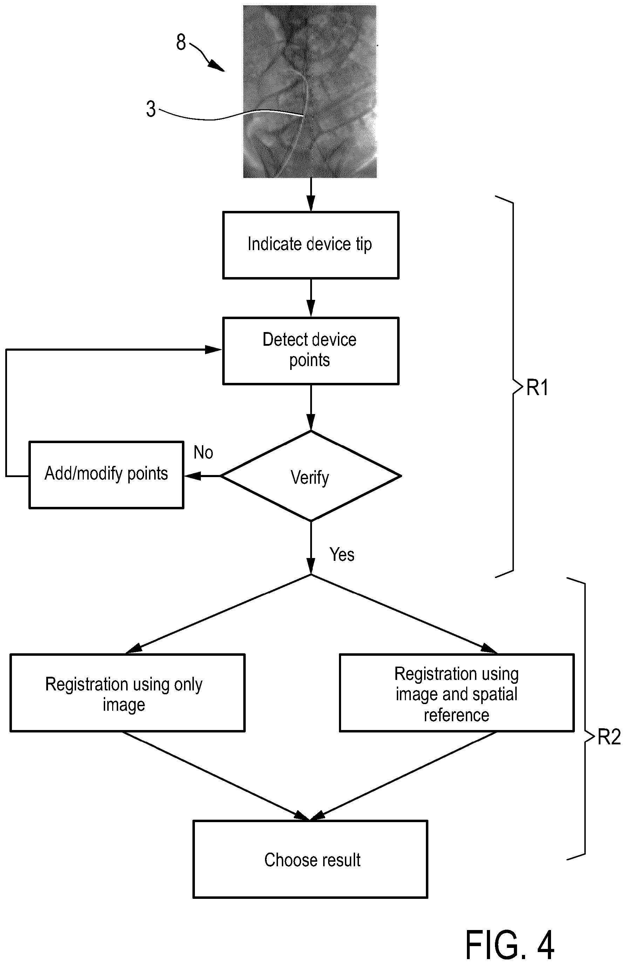

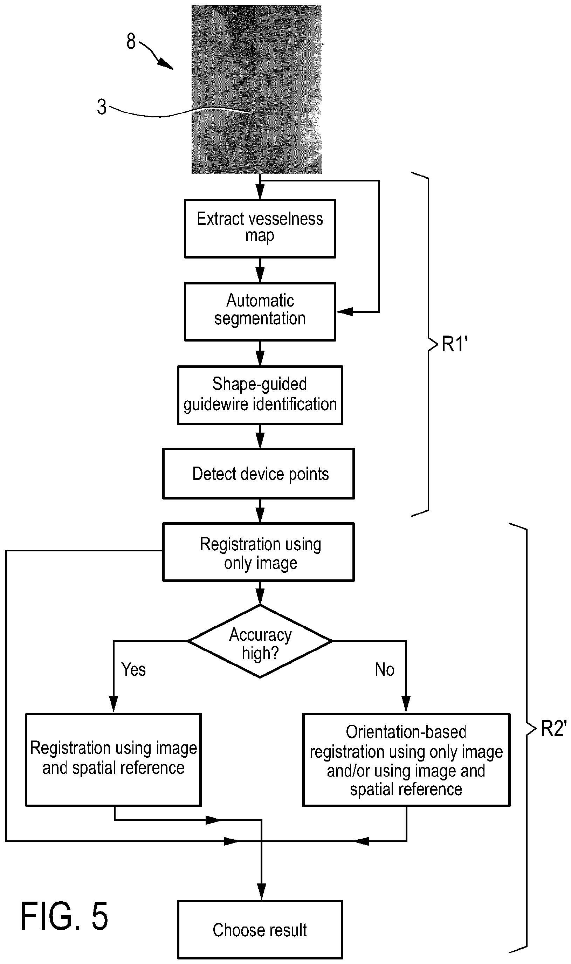

In order to ensure a good registration accuracy, particularly also when the registration is carried out automatically based on an automatic detection of the guidewire 3 in the projection image, it is preferred to validate the registration and, if necessary, resort to an alternative registration technique, wherein the alternative registration technique may particularly not use any spatial reference points for determining the respective alternative registration parameters. To illustrate this further, in the following a corresponding embodiment of a semi-automatic registration method will be exemplarily described with reference to a flow chart shown in Fig. 4, and an embodiment of a corresponding automatic registration method will be exemplarily described with reference to a flow chart shown in Fig. 5, wherein it should be understood that both registration methods may be implemented in a same product, i.e. a same software, for instance, such as by setting the automatic registration method as a default registration method, but resorting to the semi-automatic registration method if a registration accuracy resulting from the automatic registration method is found to be insufficient.

According to the semi-automatic registration method illustrated by Fig. 4, an X-ray projection image 8 showing the guidewire 3 is provided as an input. In Fig. 4, the guidewire 3 has been highlighted by a line added to the image 8, while in reality the guidewire 3, particularly its tip, will typically not be as clearly distinguishable from the rest of the image. As an additional input, coordinates of a spatial reference point may be provided. For instance, if the spatial reference point refers to a launch base for the catheter 33 as indicated above, particularly to a first tracked position of the guidewire 3, corresponding coordinates in a fixed coordinate system like the table coordinate system Σ_{Ta} may be provided based on a launch base position estimate. As indicated above, in order to confidently establish an accurate reference, the launch base position estimate can be carried out based on the coordinates of the first tracked position of the guidewire 3, which may always be set to (x, y, z) = (0, 0, 0) as indicated above, and previous registration parameters which were determined based on two or more images acquired by the imaging device 2 from different directions. For determining updated registration parameters, the registration unit 6 may then just retrieve the launch base estimate from a registration history.

The representation of the guidewire 3 in the projection image 8 is determined in a part R1 of the registration method. In this part R1, a user first indicates a tip of the guidewire 3 in the image 8 by adding a marker at an image position where he/she sees or expects the tip of the guidewire 3 to be in the image 8. In this embodiment, the user only needs to indicate the tip of the guidewire 3 in the image 8, i.e. only needs to add a single marker. However, in other embodiments, several markers may be added by the user to the image 8, thereby indicating respective several points of the guidewire 3 in the image 8. Based on the guidewire tip indicated in this embodiment as the only point corresponding to the guidewire 3 in the image 8 by the user, more proximal points of the guidewire 3 are being suggested to the user using an automatic segmentation of the image 8 and/or a calculated projection of tracked three-dimensional points of the guidewire 3 into the image 8. In particular, proximal points of the guidewire 3 in the image 8 may be determined as described in WO 2016/083275 A1, which is herewith incorporated by reference again. As indicated in Fig. 4, the user may verify the automatic detection of the more proximal points of the guidewire 3 in the image 8, wherein, if he/she is not satisfied therewith, he/she may indicate further points of the guidewire 3 in the image 8 by adding further markers to the image, or may modify existing ones by, for instance, dragging existing markers associated with automatically detected points of the guidewire 3 in the image 8 to new image positions. Once the user is satisfied, i.e. once he/she considers the guidewire points in the image 8 to be sufficient and sufficiently accurate, the guidewire points may be collectively assumed as the representation of the guidewire 3 based on which the registration parameters are to be determined. Only optionally, a more complete representation of the guidewire 3 may be determined, such as by use of a segmentation map or a minimum cost path as indicated further above. Alternatively, a representation of the guidewire 3 of this kind is already provided in the image 8 while the user still adds and/or modifies the guidewire points in the image 8. Determining the registration parameters based on only selected image points of the guidewire can allow for a registration that is more robust against possible guidewire identification inaccuracies caused by visual obstructions of one or more guidewire parts by other objects in the image.

Based on the representation of the guidewire 3, registration parameters defining the registration of the tracking device 3, 4 with the imaging device 2 are determined in a second part R2 of the registration method. The second part R2 involves determining two candidate sets for the registration parameters, wherein first candidate registration parameters are determined using both image and context information in the form of the spatial reference point (as indicated in the right block of the second part R2 in the flowchart of Fig. 4), and second candidate registration parameters are determined using only image information (as indicated in the left block of the second part R2 in the flowchart of Fig. 4).

Hence, the second candidate registration parameters are determined based on the tracked positions of the guidewire 3 in the field of view of the imaging device 2 as described by the coordinates (x, y, z) of the coordinate system Σ_{Tr}, and on the positions of the representation of the guidewire 3 in the image 8 as described by corresponding image coordinates. In particular, the second candidate registration parameters are determined by calculating two-dimensional projections of the tracked guidewire 3 under consideration of a projection geometry used by the imaging device 2 for generating the image 8, and by calculating a spatial transformation T_{Tr→Im} between the coordinate system Σ_{Tr} defined by the tracking device 3, 4 and the coordinate system Σ_{Im} defined by the imaging device 2 for which a non-combined deviation function is minimized, wherein the non-combined deviation function is defined such that it increases with a deviation between the calculated two-dimensional projection of the tracked guidewire 3 and the representation of the guidewire 3 in the image 8 as resulting from the previous part R1 of the registration method.

Thus, the second candidate registration parameters are determined based on a deviation defined only in image space. On the other hand, the first candidate registration parameters are determined as described above based on a combined deviation function that combines a deviation in image space with a deviation in physical space, the latter referring to a change of coordinates of the spatial reference point as transformed using respective registration parameters. Otherwise, however, the way in which the first candidate registration parameters are determined and the way in which the second candidate registration parameters are determined may be regarded as being identical to each other. Since in both cases several projections of the tracked guidewire 3 into the image space are calculated for finding a minimum of a deviation function indicative at least in part of a deviation between the respective calculated projection of the guidewire 3 and its representation in the image 8, the process of finding the registration parameters could also be referred to as an "aligning" of the calculated projection of the tracked guidewire 3 with its image.

In principle, the manner in which the second candidate registration parameters are determined is known from WO 2015/010859 A1, which is herewith incorporated by reference again. However, like the first candidate registration parameters, the second candidate registration parameters are now determined based on only a single image 8. As compared to a use of at least two images from different directions as described in WO 2015/010859 A1, a use of only a single image is expected to lead to a reduced registration accuracy due to a lack of depth information, i.e. information out of the image plane. Nevertheless, the second candidate registration parameters are used as a benchmark to evaluate context-based registration, i.e. registration based on the additional spatial reference point, since the image-only registration can be assumed to at least lead to an accurate in-view alignment, i.e. an accurate alignment of the calculated projection of the tracked guidewire 3 with its image, i.e. in the image 8.

The determination of the first and the second candidate registration parameters is preferably carried out iteratively, wherein the iteration preferably comprises determining successively more accurate versions of the respective registration parameters, as measured by the respective deviation measure. In this case, for both the first and the second candidate registration parameters, the minimization of the respective deviation function carried out over the several iterations, and hence the associated projections, to find the respective "optimal" registration parameters can use one or a combination of simulated annealing and Powell's method, for instance.

Already the evaluation of the respective deviation function in each step of the respective iteration can be regarded as an assessment of registration confidence (or accuracy). Moreover, however, a confidence (or accuracy) into each of the resulting first and second candidate registration parameters can be assessed.

In order to decide which of the first and the second candidate registration parameters are actually to be used, i.e. to be provided as an output of the registration method, first and second registration accuracies are determined, respectively. The first registration accuracy is determined based on the tracked guidewire 3 in the field of view of the imaging device 2, the representation of the guidewire 3 in the image 8 and the first candidate registration parameters by retrieving the minimized value of the combined deviation function, i.e. the value which the combined deviation function has for the spatial transformation between Σ_{Tr} and Σ_{Im} defined by the found first candidate registration parameters. Likewise, the second registration accuracy is determined based on the tracked guidewire 3 in the field of view of the imaging device 2, the representation of the guidewire 3 in the image 8 and the second candidate registration parameters by retrieving the minimized value of the non-combined deviation function, i.e. the value which the non-combined deviation function has for the found second candidate registration parameters.

It may be preferred that the first registration accuracy is allowed to be less than the second registration accuracy, i.e. the first candidate registration parameters are selected as output to be provided even if the first registration accuracy is less than the second registration accuracy. In particular, the first candidate registration parameters are selected as output for the registration method if and only if a) the first registration accuracy is higher than a predefined minimum registration accuracy, and b) an absolute difference between the first registration accuracy and the second registration accuracy is less than a predefined registration accuracy deviation. In this case, the first registration accuracy does preferably not correspond to the minimized value of the combined deviation function, but instead to a value of the non-combined deviation function for the projection associated with the first candidate registration parameters.

Hence, it may be preferred to compare the image and spatial context-based registration result with the purely image-based registration result in terms of an accuracy measure defined only in the image 8. In particular, then, the first registration parameters may be selected as output for the registration method if and only if a) a deviation between i) the calculated projection of the tracked guidewire 3 for which the combined deviation function was minimized and which thus led to the first candidate registration parameters, and ii) the representation of the guidewire 3 in the image 8 is less than a predefined maximum in-image deviation, and b) the difference between i) the deviation between the calculated projection of the tracked guidewire 3 for which the combined deviation function was minimized and which thus led to the first candidate registration parameters, and the representation of the guidewire 3 in the image 8, and ii) the deviation between the calculated projection of the tracked guidewire 3 for which the non-combined deviation function was minimized and which thus led to the second candidate registration parameters, and the representation of the guidewire 3 in the image 8, is less than a predefined maximum in-image deviation difference.

Allowing the image and spatial context-based registration to lead to a slightly worse alignment of the calculated projection and the representation of the guidewire 3 in the image than the purely image-based registration has been found to be reasonable because the former uses an optimization in which a compromise between in-image requirements and out-of-image requirements has to be found, whereas the latter can already focus on in-image alignment during optimization. At the same time, if the in-image alignment resulting from the first candidate registration parameters becomes too large, this is not allowed and the second candidate registration parameters are selected as output of the registration method.

Fig. 5 illustrates an automatic registration method. As indicated in Fig. 5, the same input to the method is provided as in the semi-automatic case of Fig. 4, i.e. the two-dimensional X-ray projection image 8 showing the guidewire 3 inserted into a region inside a patient's body. Again, the guidewire 3 is highlighted in terms of a relatively well visible line in Fig. 5, while in reality the visibility of the guidewire 3 in the image 8 would typically not be as good.

The automatic registration method differs from the semi-automatic registration method described with reference to Fig. 4 both in the first part relating to how the representation of the guidewire 3 in the image 8 is determined, as well as in the second part relating to how the registration parameters are determined based thereon. The first and the second part of the automatic registration method are therefore referred to by R1' and R2', respectively.

In the first part R1' of the automatic method, an automatic determination of points in the image 8 corresponding to the guidewire 3 is carried out based on a) a vesselness image, or map, determined based on the image 8, b) a segmentation of the guidewire 3 in the image 8 based on the vesselness image and the image 8 by use of an accordingly trained artificial intelligence, and c) the tracked guidewire 3 in the field of view of the imaging device 2. Further details regarding the automatic detection of the guidewire points in the image 8 are disclosed in WO 2016/083275 A1 and US 2022/0101034 A1, which are herewith incorporated again by reference. Based on the automatically determined guidewire points in the image 8, a more complete representation of the guidewire 3 in the image 8 may be determined as in the semi-automatic case, i.e., for instance, by determining a minimum cost path along the guidewire points.

The automatically determined guidewire points in the image 8 are often not pixel-wise accurate, such that the representation of the guidewire 3 in the image 8 will typically not be as accurate as, for instance, it would be possible by having a user verify the automatically detected guidewire points. But, it has been found that the automatic detection of guidewire points in the image is often accurate enough, and hence sufficient for the registration.

In the second part R2' of the automatic registration method, the representation of the guidewire 3 in the image 8 as resulting from the first part R1' of the method is validated by determining validation registration parameters based on the tracked position of the guidewire 3 in the field of view of the imaging device 2 and on the position of the representation of the guidewire 3 in the image 8, and by determining a validation registration accuracy based on the tracked position of the guidewire 3 in the field of view of the imaging device 2, the representation of the guidewire 3 in the image 8 and the validation registration parameters. In particular, the validation registration parameters and the validation registration accuracy are determined like the second candidate registration parameters and the second registration accuracy in the above described semi-automatic case, i.e. based on image information, but without using the spatial reference point.

If the validation registration accuracy satisfies a predefined criterion indicative of a satisfactory representation of the guidewire 3 in the image 8, i.e. if it can be concluded that the automatic detection of guidewire points in the image 8 was accurate enough, and preferably only in this case, first candidate registration parameters and a first registration accuracy are determined based on the representation of the guidewire 3 in the image 8 resulting from the first part R1' of the automatic registration method as described further above with respect to the semi-automatic registration method.

If, on the other hand, the validation registration accuracy satisfies a predefined criterion indicative of an unsatisfactory representation of the guidewire 3 in the image 8, i.e. if it is concluded that the automatic detection of guidewire points in the image 8 was not sufficiently accurate, further candidate registration parameters and a corresponding further registration accuracy are determined.

The predefined criterion indicative of a satisfactory representation of the guidewire 3 in the image 8 and the predefined criterion indicative of an unsatisfactory representation of the guidewire 3 in the image 8 are preferably complementary to each other, i.e. one is satisfied if the other is not and vice versa. For instance, a predefined minimum accuracy corresponding to a predefined maximum value of the non-combined deviation function referred to further above regarding the semi-automatic case may be considered, wherein if the validation registration accuracy is equal to or higher than this predefined minimum registration accuracy, the first candidate registration parameters are determined, i.e. registration parameters are determined by using additionally the spatial reference point, and if the validation registration accuracy is less than the predefined minimum registration accuracy, the further candidate registration parameters are determined.

If determined at all, the further candidate registration parameters are determined based on the tracked guidewire 3 in the field of view of the imaging device 2, on the representation of the guidewire 3 in the image and additionally on orientations associated with the representation of the guidewire 3 in the image, but preferably, although not necessarily, not on any spatial reference point. In particular, the further candidate registration parameters are determined by minimizing a further combined deviation function, the further combined deviation function being defined such that it increases with a) a deviation between the calculated projection of the tracked guidewire 3 and its representation in the image 8, and b) a deviation between orientations associated with the calculated projection of the tracked guidewire 3 in the image 8 and the orientations associated with the representation of the guidewire 3 in the image 8.

If the further registration parameters are also determined based on the at least one spatial reference point, the further combined deviation function may further increase with the deviation between i) coordinates of the at least one spatial reference point determined based on respective registration parameters considered during the minimization and ii) coordinates of the at least one spatial reference point considered as accurate, such as due to being determined based on previous registration parameters taken as reference for a registration update. In a variant, two sets of further registration parameters may be determined, wherein a first set of further registration parameters is determined without using any spatial reference point and the second set of further registration parameters is determined based additionally on at least one spatial reference point.

Moreover, for determining the further candidate registration parameters, the tip of the guidewire 3 in its representation in the image 8 is neglected. As described further above, this is because the tip of an elongate object such as a guidewire 3 in images like X-ray images tends to be the point most difficult to detect for automatic detection techniques, and hence it can be assumed that a wrongly detected guidewire tip was most likely the reason for the unsatisfactory validation registration accuracy. The lack of the guidewire tip is attempted to be compensated by use of the orientations of the representation of the guidewire 3 in the image 8 at its remaining points in determining the further candidate registration parameters. Also for the further candidate registration parameters a registration accuracy, i.e. a further registration accuracy, is determined, namely based on the tracked guidewire 3 in the field of view of the imaging device 2, on the representation of the guidewire 3 in the image 8 with the guidewire tip removed, and on the further candidate registration parameters. In particular, the further registration accuracy is in this case set to be the found minimum value of the further combined deviation function, i.e. the value of the combined deviation function corresponding to the further candidate registration parameters.

Depending on whether the validation registration accuracy was satisfactory or not, one arrives at either one or two registration results to choose from for the output of the automatic registration method (assuming a single set of further registration parameters being determined). In any case, one registration result has been arrived at without reference to orientations of the representation of the guidewire 3 in the image 8. If the validation registration accuracy was unsatisfactory, this registration result corresponds to the validation registration parameters and hence essentially the second candidate registration parameters. If the validation registration accuracy was satisfactory, this first registration result is determined based on the validation registration parameters and the first candidate registration parameters as described further above in the semi-automatic case. Additionally, a second registration result has been arrived at in case the validation registration accuracy was insufficient and hence further candidate registration parameters were determined. Hence, the second registration result to choose from corresponds to the further candidate registration parameters, which are preferably determined without use of any spatial reference point.

To determine the registration parameters defining the registration of the tracking device 3, 4 with the imaging device 2, i.e. to choose "the best" of the two sets of registration parameters to be provided as output by the automatic registration method, respective registration accuracies are measured in terms of a further accuracy measure and compared to each other. The further accuracy measure is defined on any two-dimensional projection of the guidewire 3 into the image 8 calculated by some registration parameters with respect to a given representation of the guidewire 3 in the image 8 by a sum of four terms defined as follows.

The first term, which is herein referred to as averageDistance, is calculated by determining, for each of the image points of the calculated projection, a distance to the respective nearest image point corresponding to the representation of the guidewire 3, and subsequently determining an average of the distances determined for all points of the calculated projection.

The second term, which is herein referred to as orientationDistance, is calculated by determining, for each of the image points of the calculated projection, a difference in orientation of the projection relative to an orientation of the representation of the guidewire 3, and by determining an average of the calculated differences in orientation over all image points of the calculated projection.

Moreover, two further terms, which are herein referred to as outlierRate and outlierRateLarge, are calculated. The quantity outlierRate is calculated by determining, for all distances calculated for determining the quantity averageDistance, a ratio above a first predetermined threshold which is preferably equal to 0.5 mm. For determining the quantity outlierRateLarge, a ratio among the distances used for calculating the quantity averageDistance is determined that lie above a second predefined threshold which higher than the first predetermined threshold and preferably equal to 1.0 mm.

As mentioned above, any spatial reference point can be used for determining the registration parameters based additionally thereon, in particular any point to which coordinates are associated by each of the coordinate systems considered, i.e. the coordinate system defined by the tracking device 3, 4, the coordinate system defined by the imaging device 2 and, if used, the coordinate system defined by the table 26 or another fixed object. Moreover, as also mentioned above, several spatial reference points may be used in combination for determining the registration parameters based additionally thereon. Preferred choices for the spatial reference points will subsequently be described in more detail.

Fig. 6 illustrates schematically and exemplarily the use of a docking top 81 for "launching" a guidewire 3 of a catheter 33. The docking top comprises a frame via which it is fixed to an operation table like the table 26 shown in Fig. 1, and a slot module within this case three slots via which the guidewire 3 can be "launched", wherein the slot module can be held fixed by the frame. As indicated in Fig. 6, before fixing the slot module to the frame, a surgical drape is laid over the patient and the frame. The "launching" of the guidewire 3 refers to its insertion from outside of the region covered by the surgical drape to below the surgical drape via one of the slots in the slot module. As the "nose" of the frame shown in Fig. 6 indicates, the three slots of the slot module may have different proximal openings, but can end in a same distal opening, wherein the same distal opening of the three slots corresponds in its position to a hole in the frame and a corresponding hole in the surgical drape. The guidewire 3 is typically inserted into one of the openings by use of a docking fin, which could also be regarded as an injection module and serves as an instrument base for the guidewire 3.

The "launching" of the guidewire 3 through one of the slots of the slot module provides a physical constraint for the guidewire 3, since it is constrained in its physical movement, i.e. in how it can translate and/or rotate in one or more spatial directions, by being led through the respective slot. In an example, the point where the guidewire 3 enters the respective slot of the slot module, which could be regarded as a "launch base" for the guidewire 3, is taken to be the spatial reference point used for determining the registration parameters. In another embodiment, another point related to a location of the slot via which the guidewire 3 is led could be chosen as the spatial reference point.

As mentioned above, the registration carried out preferably refers to an updated registration after a previous registration, such that the registration parameters defining the updated registration are updated with respect to previous registration parameters. While the updated registration parameters are to be determined based on a single image of the guidewire 3 in the field of view of the imaging device 2 by use of the "launch base" point, the previous registration parameters may particularly still have been determined without use of a spatial reference point, but based on two images of the guidewire 3 in the field of view of the imaging device 2 from different directions, in order to increase the chances that a resulting registration accuracy in depth, i.e. in a direction out of the image plane, remains acceptable after the updated registration based on the single image. During the previous, "two-view" registration, the coordinates of the "launch base" point in the coordinate system defined by the table 26 are computed, wherein it is understood that, in another embodiment, another fixed coordinate system could be used in place of the coordinate system defined by the table. Additionally or alternatively, the "launch base" point is mapped, during the re-registration but by use of the previous registration parameters, from the coordinate system defined by the tracking device 3, 4 to the coordinate system defined by the imaging device 2, wherein the coordinate system defined by the tracking device 3, 4 is defined such that it assigns the "launch base" point the coordinates (0, 0, 0) of its origin. If a registration history is stored that includes more than one two-view registration results, i.e. more than one set of previous registration parameters determined based on at least two images of the guidewire 3 in the field of view of the imaging device 2, the most recent set of these is used for estimating the "launch base" point, since it is expected to provide the most up-to-date and hence most accurate estimate.

Which of the slots of the slot module is currently in use, i.e. into which of the slots the guidewire 3 is currently inserted, can be tracked. Hence, it can be determined whether the "launch base" point has changed, such as due to a change of slot, and, if so, the updated registration parameters can be determined on the changed "launch base" point. For each registration result stored in the registration history, a corresponding slot which was in use at the respective time may be stored.

Fig. 7 illustrates schematically and exemplarily further choices for the spatial reference point. According to these choices, the spatial reference point refers to an insertion opening 82 through which the guidewire 3 and subsequently the catheter 33 are inserted into a patient's body. The insertion is carried out via a vessel, such as in the groin for femoral access or in the arm for brachial access to the heart, for instance. Such insertion points are typically stable and often present on both sides of the body, i.e. right and left.

The coordinates of the actually used insertion point 82 in the coordinate system defined by the tracking device 3, 4 can be determined based on measured characteristics such as a curvature of the guidewire 3, wherein the thus determined coordinates are mapped to a fixed coordinate system such as the one defined by the table 26 by use of the previous registration parameters resulting from a two-view registration. Alternatively, if stored for the previous registration, the coordinates of the spatial reference point 82 in the fixed coordinate system may be loaded from a registration history. Like the "launch base" point, also an insertion opening can be referred to in terms of a single point in three-dimensional space.

The registration unit 6 may be configured to check whether an insertion point 82 that is currently in use corresponds to the insertion point 82 based on which the previous registration has been carried out. In particular, the registration unit 6 may be configured to check whether coordinates of an insertion point 82 currently in use in the fixed coordinate system are available via a) computation based on its coordinates in the coordinate system of the tracking device 3, 4 as currently sensed and the previous registration parameters or b) access to the registration history. If this is not the case, the registration unit 6 may be configured to exclude the insertion point 82 from use as spatial reference point for determining the updated registration parameters. Instead, one or more other spatial reference points may be used. Such a check for a viability of an insertion point 82 currently in use as a spatial reference point can be particularly relevant in case several different insertion points 82 are used in the course of an interventional procedure.

Further choices for the at least one spatial reference point relate to a lumen in which the guidewire 3 and catheter 33 are being moved. Positions of the lumen can be estimated, for instance, based on an image acquired using an imaging modality that has been pre-registered with the imaging device 2 to be registered with the tracking device 3, 4, particularly based on a vessel segmentation map.

A vessel segmentation map can be determined by thresholding a contrast-enhanced volumetric computed tomography (CT) image, wherein the volumetric CT image and thus the vessel segmentation can be registered to the imaging device 2. This type of vessel map is often used as a roadmap for minimally invasive procedures in many navigation solutions. For instance, the guidewire 3 can be visualized in 3D within a 3D vessel map. Because the guidewire 3 and the catheter 33 are navigated within the vessels, even though the vessel map information may not be fully accurate regarding the vessel position during the procedure, it can still be utilized to constrain the registration parameters and hence the device position calculated therefrom in single view registration.

A vessel map can be represented as a 3D volumetric segmentation map where vessels are assigned to one value and the rest to another value. Another possibility is to use a 3D mesh-based map as vessel map. Also, certain vessel positions may be selected, such as locations around ostia, wherein those selected positions may be used as spatial reference points for determining the registration parameters. In all cases, 3D vessel coordinates can either already correspond to or be mapped to the coordinates used in shape registration, such as coordinates in the coordinate system defined by the table 26, for instance.

Additionally or alternatively, positions of the lumen can be estimated, for instance, based on several stored tracking positions of the respective instrument 3, 33 after insertion into a patient's body, particularly based on a shape occupancy map.

A shape occupancy map is a 3D map similar to a vessel map. It can be computed by recording sensing data relating to the instrument moving in a patient's body over time. To calculate a shape occupancy map, a 3D position model is set up and initialized with all positions having a counter equal to zero. With each set of instrument points determined by the tracking device 3, 4 based on the sensing data, the counter of the positions of the position model is increased based on the determined instrument points. Depending on predefined settings, all determined instrument points or only distal points, such as points around a guidewire tip, or only points of the instrument segment in the body may be used to update the position model defining the shape occupancy map. Additionally or alternatively, a shape occupancy map can use temporal information and weigh more recently determined instruments points more heavily. The 3D position map is dynamically created and can be used complementary to a vessel map. It can be represented in the same way as a vessel map, i.e., for instance, as a full 3D map, in terms of vessel center lines and vessel radii, et cetera. To use it in registration, the counter values of the positions can be used as weights, or the map can be thresholded to calculate a binary map, from which center lines can be calculated. In all cases, similar to other uses, 3D coordinates of the shape occupancy map either already correspond to or are transformed to a fixed coordinate system, such as table coordinates, for use in the registration.

After the registration has been completed, the guidewire 3 can be moved within the person 25, wherein during the movement the tracking device 3, 4 can track the locations of the guidewire 3 within the person 25 and the tracked locations of the guidewire 3 can be shown in an arbitrary pre-registered volumetric image of a region of interest in the internal anatomy of the person 25, in which the guidewire 3 is actually moved, because a position determination unit 14 can determine the locations of the guidewire 3 within the volumetric image based on the tracked actual location of the guidewire 3 and the determined registration parameters. The volumetric image in which the tracked position of the guidewire 3 can be shown is also referred to herein as a navigation image or "position image". It can be, for instance, a contrast-enhanced CT image that was acquired before the intervention, wherein image points of the volumetric image have been assigned coordinates in the coordinate system defined by the imaging device 2 or another fixed coordinate system via a pre-registration. The contrast of the CT image can be used for determining the vessel map indicated above. The vessel map can therefore also be regarded as a processed version of the CT image. The tracked position of the guidewire 3 can hence also be visualized in the vessel map, as already indicated above.

Besides showing the tracked position of the guidewire 3 in a volumetric image acquired before the intervention, it can optionally also be shown in further X-ray, two-dimensional projection images acquired after the registration. Also in these images, the position determination unit 14 can determine the location of the guidewire 3 based on the tracked actual location of the guidewire 3 and the determined registration parameters. However, for navigating the guidewire, a volumetric image may be preferred.

The position determination unit 14 can be part of navigational image providing unit that also provides the "bare" navigational image, in which the subsequently the guidewire position is determined and/or a representation thereof is provided.

A data receiving and/or providing unit receiving sensing data from the tracking device 3, 4, and comprising the object detection unit 5 for receiving and/or providing object image data comprising a position and/or orientation and/or shape of the object in images generated by the image 2, can, together with the registration unit 6, be regarded as forming a registration system 13 for registering the imaging device 2 with the tracking device 3, 4. Moreover, the imaging device 2, the tracking device 3, 4, the registration system 13 and a navigational image providing unit comprising the position determination unit 14 can be regarded as forming an imaging system for imaging the guidewire 3 within the person 25.

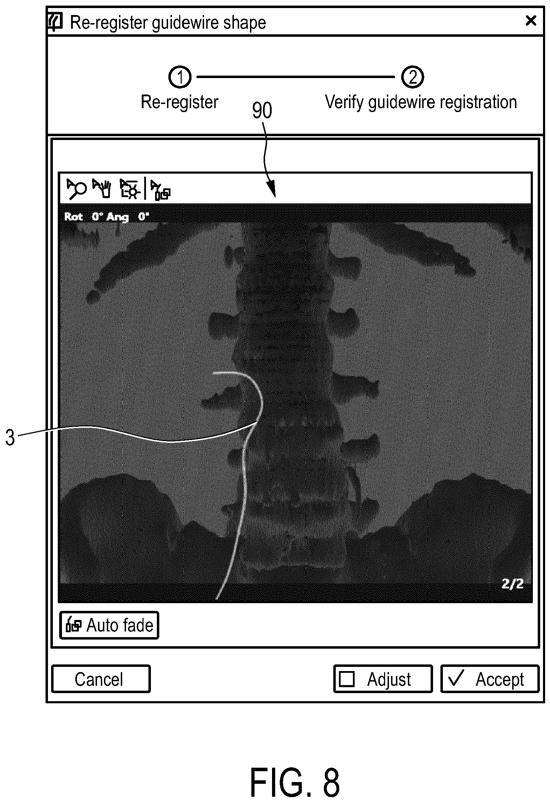

Fig. 8 shows schematically and exemplarily a graphical user interface provided by the imaging system as it may be displayed on the display 17. The graphical user interface includes a position image part corresponding to a two-dimensional view of a volumetric navigational image 90 showing a 3D skeletal anatomy of the person 25 in an abdominal region, wherein a representation of the guidewire 3 in terms of a clearly visible line has been added by the position determination unit 14. The graphical user interface furthermore comprises several elements via which a user can provide inputs. In particular, the user can indicate, by clicking on corresponding buttons, whether or not he/she is satisfied with the shown representation of the guidewire 3 and hence the registration result. Other user inputs concern the shown view on the person's anatomy, i.e. image preferences. If the user is not satisfied, the registration, which is in this case an updated registration or "re-registration", is carried out. As indicated in an upper area of the graphical user interface shown in Fig. 8, a further graphical user interface, which may also be regarded as a further window or screen of the same graphical user interface, may be provided for the registration process.

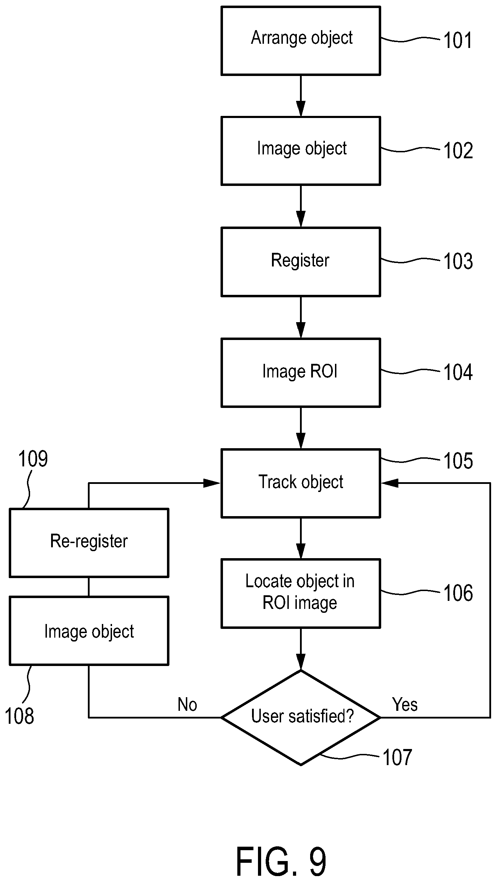

In the following an embodiment of an imaging method for monitoring an interventional procedure will exemplarily be described with reference to the flowchart shown in Fig. 9.

In a first step 101, the guidewire 3 is arranged in the field of view of the imaging device 2 and the imaging device 2 generates one or more two-dimensional projection images of the guidewire 3. Moreover, the points along the guidewire 3 within the field view of the imaging device 2 are determined by the tracking device 3, 4 based on corresponding sensing data being acquired. In a second step 102, the two-dimensional projection image is shown to the user by using the display 17, wherein the user adds one or more markers to the two-dimensional projection image for indicating a position in the two-dimensional projection image at which he/she expects or sees the tip of the guidewire 3. The object detection unit 5 may for this purpose be configured to provide a graphical user interface, which allows the user to click on an arbitrary point in the image provided by the imaging device 2, in order to add a marker. Moreover, the object detection unit 5 provides a representation of the guidewire 3 in the two-dimensional projection image based on the added marker and an automatic segmentation of more proximal parts of the guidewire 3 in the image. In a third step 103, the registration unit 6 determines initial registration parameters defining an initial registration of the tracking device 3, 4 with the imaging device 2 based on the tracked location of the guidewire 3 in the field of view of the imaging device 2 and on the location of the representation of the guidewire 3 in the two-dimensional projection image. The first, the second and the third step can be regarded as forming a registration method, which may be performed during a calibration procedure, in which the interventional system is calibrated, before the guidewire 3 is inserted into the person 25.

Additionally or alternatively to a registration carried out before the person 25 is arranged on the support means 26 within the field of view of the imaging device 2 and the guidewire 3 is introduced into the person 25, a registration can be carried out while the person 25 is arranged on the support means 26 within the field of view of the imaging device 2 and after the guidewire 3 has been introduced into the person 25. Steps 101 and 103 can then still be carried out in the same way, the difference just being that the guidewire 3 may be less clearly visible in images acquired by the imaging device 2.

After the initial registration parameters have been determined, a volumetric navigational image of a region of interest (ROI) within the person 25 that was previously acquired and is pre-registered with the imaging device 2 is provided in a fourth step 104, such as by loading it from a storage, for instance. The region of interest is preferentially the region within the person 25, through which the guidewire 3 should be moved. Then, the movement of the guidewire 3 within the person 25 is tracked by the tracking device 3, 4 in a fifth step 105. In a sixth step 106, the location of the guidewire 3 within the volumetric image, which has been provided in the fourth step, is determined based on the tracked guidewire 3 and the determined registration parameters, and the display 17 shows the determined location of the guidewire 3 within the volumetric image.

In a seventh step 107, it is checked whether the user has indicated, by a corresponding user input, whether a re-registration is to be carried out, such as because he/she is dissatisfied with the visualization of the guidewire 3 in the volumetric image. As long as this is not the case, i.e., as long as the user is satisfied, i.e. confident that the registration is still accurate, the method continues with the fifth step. In particular, the fifth and the sixth step can be continuously performed in a loop as long as the user has not indicated a need for a re-registration.

By performing the fifth and the sixth step in a loop during the movement of the guidewire 3 within the person 25, the user can monitor the movement of the guidewire 3 within the person 25 in real-time, without necessarily acquiring real-time X-ray images. However, if it is determined in step 107 that the user is not satisfied with the current registration anymore, a two-dimensional projection image of the ROI, including the guidewire 3, from a current imaging direction is acquired in a step 108, and a re-registration is carried out by the registration unit 6 by determining updated registration parameters defining an updated registration of the tracking device 3, 4 with the imaging device 2 based on the tracked guidewire 3 in the field of view of the imaging device 2 and on the representation of the guidewire 3 in the image acquired in step 109. The method then continues again with steps 105 and 106, wherein now the guidewire 3 is identified by use of the updated registration parameters in the pre-registered volumetric navigational image. Hence, the user can again accurately monitor the movement of the guidewire 3 within the person 25 in real-time, without necessarily acquiring real-time X-ray images. Moreover, since only a single image of the ROI needs to be acquired in step 108 when determining the updated registration parameters as described above by also using a spatial reference point as a basis, the re-registration is simplified.

The registration procedure described herein is not limited to a certain imaging modality. Thus, although in the above described embodiments the imaging device is an X-ray C-arm device, in other embodiments also other imaging devices can be registered with the tracking device like other radiographic imaging devices, ultrasound imaging devices, magnetic resonance imaging devices, et cetera. Even one or more cameras could in principle be used for the registration.

Moreover, the image in which the respective object is being shown while the object is navigated based on the registration, i.e., the "position" or "navigational" image, is not limited to any specific image type. In particular, it is not limited to being a CT image or a vessel or shape occupancy map derived from a CT image. As already indicated, it is not even necessarily three-dimensional, but could also be, for instance, a two-dimensional projection image acquired by the X-ray C-arm device or a different two-dimensional image registered with the X-ray C-arm device.

Furthermore, as far as the above embodiments related to projections of an assumed transformed three-dimensional position and/or orientation and/or shape of an object as indicated by sensing data, into a two-dimensional image used for registration, wherein the respective assumed transformation related to a coordinate transformation defined by a respective version of registration parameters, same or similar registration results may also be achieved with alternative calculations. In particular, instead of determining in-image deviations between the respective projections and a representation of the object indicated by object image data, corresponding deviations may also be determined between i) respective inversely transformed back-projections of the representation of the object indicated by the object image data and ii) the non-transformed three-dimensional position and/or orientation and/or shape of the object as indicated by the sensing data. For any given, or assumed, version of registration parameters, a respective inversely transformed back-projection of the representation of the object indicated by the object image data may be determined by back-projecting the representation from the image into three-dimensional space and then transforming the result of the back-projection from a coordinate system defined by the imaging device to a coordinate system defined by the sensing device. In this way, instead of assessing a change across several projections of the object into an image with respect to a single representation of the object in the image, a change of various three-dimensional positions associated with the object image data would be assessed with respect to a single representation of the object given by the sensing data. In both cases, the respective change can be considered as a parameter for an assessment of registration confidence (or accuracy), such as in terms of respective deviation measures, for instance.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the acquiring of sensing data, particularly the tracking of an object, the receiving and/or providing of data like sensing data or objection image data, particularly the providing of a representation of an object in an image, the determining of registration parameters, et cetera, performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a registration system for registering an imaging device for generating two-dimensional images of an object with a sensing device for acquiring sensing data indicative of a three-dimensional position and/or orientation and/or shape of the object. Besides the sensing data, object image data comprising a position and/or orientation and/or shape of the object in an image generated by the imaging device are received and/or provided in the system, wherein the system comprises a registration unit configured to determine a registration between the sensing device and the imaging device based on i) the object image data, ii) the sensing data and iii) an assessment of registration confidence (or accuracy), wherein said determined registration is within a predetermined acceptable confidence (or acceptable accuracy). This can allow for a faster, but still accurate registration, particularly based on only a single image of the imaging device.

## Claims

1. A registration system (13) for registering an imaging device (2) for generating two-dimensional images (7, 8) of an object (3) with an elongated sensing device (3, 4) arranged for acquiring sensing data indicative of a three-dimensional position and/or orientation and/or shape of the object (3), the registration system (13) comprising:
- a data receiving and/or providing unit configured to receive or provide i) object image data comprising a position and/or orientation and/or shape of the object (3) in an image (7, 8) generated by the imaging device (2), and to receive or provide ii) the sensing data acquired by the sensing device (3, 4), and
- a registration unit (6) configured to determine registration parameters defining a registration between the sensing device (3, 4) and the imaging device (2) based on i) the object image data, ii) the sensing data and iii) an assessment of registration confidence wherein said determined registration is within a predetermined acceptable confidence.

2. The registration system (13) as defined in claim 1, wherein the data receiving and/or providing unit is further configured to receive and/or provide context data indicative of a position of at least one spatial reference point (80, 80') whose change with registration parameters is a parameter for the assessment of registration confidence.

3. The registration system (13) as defined in claim 2, wherein a change of various three-dimensional positions of object image data is another parameter for the assessment of registration confidence.

4. The registration system (13) as defined in claim 2 or 3, wherein the context data are indicative of a) a position of the at least one reference point (80, 80') in a three-dimensional coordinate system defined by the imaging device (2), and at least one of b) a position of the at least one spatial reference point (80, 80') in a three-dimensional coordinate system defined by the sensing device (3, 4) and b') a position of one or more points of the object (3) associated with the at least one spatial reference point (80, 80') in a three-dimensional coordinate system defined by the sensing device (3, 4).

5. The registration system (13) as defined in any of claims 2 to 4, wherein the registration unit (6) is configured to determine the registration parameters based on i) a first deviation determined based on the object image data and the sensing data, and on ii) at least one second deviation determined based on the context data.

6. The registration system (13) as defined in any of previous claims, wherein the registration parameters determined by the registration unit (6) are updated registration parameters defining an updated registration of the sensing device (3, 4) with the imaging device (2) after a previous registration of the sensing device (3, 4) with the imaging device (2) defined by previous registration parameters.

7. The registration system (13) as defined in claims 5 and 6, wherein the registration unit (6) is configured to determine the at least one second deviation based on i) the context data and ii) the previous registration parameters.

8. The registration system (13) as defined in claim 7, wherein the registration unit (6) is configured to determine several versions of updated registration parameters, wherein the determination of each version comprises i) calculating an assumed spatial transformation between the coordinate system defined by the sensing device (3, 4) and the coordinate system defined by the imaging device (2), and ii) calculating, based on the sensing data, a two-dimensional projection of the object (3) on the plane of image (7, 8) to which the object image data relate,
wherein the registration unit (6) is configured to select the updated registration parameters defining the registration of the sensing device (3, 4) with the imaging device (2) based on values of the first deviation and the second deviation for the several determined versions of updated registration parameters.

9. The registration system (13) as defined in any of claims 2 to 8, wherein the registration unit (6) is further configured to:
- determine first candidate registration parameters based on i) the object image data, ii) the sensing data and iii) the context data, and to determine a first registration accuracy based on i) the object image data, ii) the sensing data and iii) the first candidate registration parameters,
- determine second candidate registration parameters based on i) the object image data and ii) the sensing data without using the context data, and to determine a second registration accuracy based on i) the object image data, ii) the sensing data and iii) the second candidate registration parameters, and
- determine the registration parameters defining the registration of the sensing device (3, 4) with the imaging device (2) based on one or a weighted combination of the first candidate registration parameters and the second candidate registration parameters based on the first registration accuracy and the second registration accuracy.

10. The registration system (13) as defined in any of claims 2 to 9, wherein the registration unit (6) is configured to:
- determine first candidate registration parameters based on i) the object image data, ii) the sensing data and iii) the context data,
- determine further candidate registration parameters based on i) the object image data and ii) the sensing data, wherein the object image data comprise several image points and associated orientations corresponding to the object (3), wherein the orientations are indicative of directions of straight lines between the several image points, and
- determine the registration parameters defining the registration of the sensing device (3, 4) with the imaging device (2) based on the first candidate registration parameters, the further candidate registration parameters, and a registration accuracy determined for each of the first candidate registration parameters and the further candidate registration parameters.

11. The registration system (13) as defined in any of claims 2 to 10, wherein the object (3) is an interventional medical instrument (33), and wherein the at least one spatial reference point (80, 80') refers to any of the following:
- an instrument base (81) fixed in the image coordinate system,
- a determined insertion point (82) through which the instrument (33) is inserted into a patient's body, and
- a point of a lumen in a patient's body in which the instrument (33) is movable, the position of the point of the lumen being provided a) based on an image acquired using an imaging modality that has been pre-registered with the imaging device (2) to be registered with the sensing device (3, 4) and/or b) based on several stored positions of the instrument (33) after insertion into a patient's body.

12. An imaging system for imaging an object (3), the imaging system comprising:
- an imaging device for generating an image of a region of interest,
- a sensing device (3, 4) for acquiring sensing data indicative of a three-dimensional position and/or orientation and/or shape of the object (3) in the region of interest,
- a registration system (13) as defined in any of the preceding claims for determining registration parameters defining a registration of the sensing device (3, 4) with the imaging device, and
- a navigational image providing unit for providing a navigational image of the region of interest including a representation of the object based on the sensing data and the determined registration parameters.

13. An interventional system (1) comprising:
- an interventional instrument (33) for carrying out an interventional procedure, and
- an imaging system for imaging the interventional instrument (33) as defined in claim 12.

14. A registration method for registering an imaging device (2) for generating two-dimensional images (7, 8) of an object (3) with an elongated sensing device (3, 4) arranged for acquiring sensing data indicative of a three-dimensional position and/or orientation and/or shape of the object (3), the registration method comprising:
- receiving and/or providing i) object image data comprising a position and/or orientation and/or shape of the object (3) in an image (7, 8) generated by the imaging device (2), and ii) the sensing data acquired by the sensing device (3, 4), and
- determining registration parameters defining a registration between the sensing device (3, 4) and the imaging device (2) based on i) the object image data, ii) the sensing data and iii) an assessment of registration confidence wherein said determined registration is within a predetermined acceptable confidence.

15. A registration computer program for registering an imaging device (2) for generating two-dimensional images (7, 8) of an object (3) with an elongated sensing device (3, 4) arranged for acquiring sensing data indicative of a three-dimensional position and/or orientation and/or shape of the object (3), the registration computer program comprising program code means for causing a registration system (13) as defined in any of claims 1 to 11 to carry out the steps of the registration method as defined in claim 14 when the registration computer program is run on a computer controlling the registration system (13).
